# FASCICULE DE BREVET EUROPEEN

(11) **EP 1 137 804 B1**
(45) Date de publication et mention de la délivrance du brevet: **18.02.2004**
(21) Numéro de dépôt: 99958276.0
(22) Date de dépôt: 08.12.1999
(51) Int. Cl.: C12Q 1/68

(54) **METHODE DE DETECTION IN VITRO D'UNE SUBSTANCE CABLE DANS UN ECHANTILLON COMPRENANT LE MARQUAGE DE LADITE SUBSTANCE PAR UN GENE RAPPORTEUR ET PAR LES SEQUENCES NECESSAIRES A L'EXPRESSION DUDIT GENE RAPPORTEUR IN VITRO**
KENNZEICHNUNG EINER SUBSTANZ MITTELS EINES REPORTERGENS UND SEQUENZEN, DIE FÜR DIE IN VITRO EXPRESSION DES REPORTERGENS NOTWENDIG SIND
METHOD FOR DETECTING IN VITRO A TARGET SUBSTANCE IN A SAMPLE COMPRISING THE LABELLING OF SAID SUBSTANCE WITH A REPORTER GENE AND THE SEQUENCES REQUIRED FOR EXPRESSING SAID REPORTER GENE IN VITRO

(30) Priorité: 08.12.1998 FR 9815489
(43) Date de publication de la demande: 04.10.2001
(73) Titulaire: Proteus S.A., 30000 Nîmes (FR)
(72) Inventeur: Dautel, Sandrine, 33000 Nîmes (FR); Persillon, Cécile, 30000 Nîmes (FR); Dupret, Daniel, 30420 Calvisson (FR); Masson, Jean-Michel, 31400 Toulouse (FR); Lefevre, Fabrice, 30900 Nîmes (FR)
(74) Mandataire: Breese, Pierre
(86) Numéro de dépôt international: PCT/FR1999/003061
(87) Numéro de publication internationale: WO 2000/034513

(56) Documents cités:
- EP-A- 0 518 557
- WO-A-92/07949
- WO-A-94/03630
- WO-A-94/05812
- WO-A-94/06928
- WO-A-94/24303
- WO-A-96/01327
- WO-A-96/08580
- WO-A-97/19193
- WO-A-98/11249
- WO-A-98/27225
- GB-A- 2 276 621
- SUN Y ET AL: "COMPARISON OF PEPTIDE ENZYME-LINKED IMMUNOSORBENT ASSAY AND RADIOIMMUNOPRECIPITATION ASSAY WITH IN VITRO-TRANSLATED PROTEINS FOR DETECTION OF SERUM ANTIBODIES TO HUMAN PAPILLOMAVIRUS TYPE 16 E6 AND E7 PROTEINS" JOURNAL OF CLINICAL MICROBIOLOGY,US,WASHINGTON, DC, vol. 32, no. 9, 1 septembre 1994 (1994-09-01), pages 2216-2220, XP000610176 ISSN: 0095-1137
- HANES J & PLÜCKTHUN A: "IN VITRO SELECTION AND EVOLUTION OF FUNCTIONAL PROTEINS BY USING RIBOSOME DISPLAY" PROCEEDINGS OF THE NATIONAL ACADEMY OF SCIENCES OF USA, vol. 94, mai 1997 (1997-05), pages 4937-4942, XP002077149
- RESTO ET AL.: "AMPLIFICATION OF PROTEIN EXPRESSION IN A CELL FREE SYSTEM" NUCLEIC ACIDS RESEARCH, vol. 20, no. 22, 1992, pages 5979-5983, XP002119012
- CASSATA G ET AL: "Rapid expression screening of Caenorhabditis elegans homeobox open reading frames using a two-step polymerase chain reaction promoter -gfp reporter construction technique" GENE: AN INTERNATIONAL JOURNAL ON GENES AND GENOMES, vol. 212, no. 1, 28 mai 1998 (1998-05-28), page 127-135 XP004122433 ISSN: 0378-1119
- HIRAYAMA ET AL: 'Evalutaion of Antioxidant Activity by Chemiluminescence' ANALYTICAL BIOCHEMISTRY vol. 247, 1997, pages 237 - 241

## Description

La présente invention concerne une méthode de détection *in vitro* d'une substance cible, notamment une séquence nucléique mais plus généralement tout type de substance, dans un échantillon. La recherche de substances cibles, notamment de séquences d'acides nucléiques, représente un objectif majeur dans de nombreux laboratoires de recherche impliqués dans de nombreux domaines d'activité, et principalement dans le domaine médical ou agroalimentaire. Dans ces domaines, la recherche de séquences cibles vise par exemple :
- Le diagnostic de virus à l'origine de maladies, telles que le Sida (HIV) ou l'hépatite B (HBV).
- Le diagnostic spécifique de maladies d'origine bactérienne, telle que la tuberculose ou la lèpre.
- Le diagnostic de mutations à l'origine de maladies génétiques ou de cancers cellulaires.
- Le diagnostic d'une contamination bactérienne dans une chaîne agroalimentaire.
- La recherche des microorganismes impliqués dans la corrosion biologique des canalisations ou des containers utilisés dans des procédés industriels.

La difficulté majeure des méthodes de diagnostic utilisées dans l'art antérieur réside dans la spécificité, la sensibilité, la rapidité et la reproductibilité du test de détection utilisé. Ces difficultés proviennent généralement de la nature du marquage utilisé. En effet, la nature du marquage d'une substance est le point décisif de toute détection ultérieure permettant de suivre, ou quantifier ladite substance. Qu'il s'agisse d'un diagnostic humain, animal ou végétal, en agroalimentaire, en thérapie, en pharmacologie, en recherche, dans des procédés industriels variés etc...., il est nécessaire de détecter, de suivre et de quantifier spécifiquement une ou plusieurs substances cibles. Pour que cette détection soit optimale, il est nécessaire de disposer de techniques de marquage performantes et sensibles.

Une des techniques de marquage spécifiques d'acides nucléiques utilise l'amplification par PCR. Le marquage d'amorces utilisables en PCR peut se faire de deux façons, soit par le marquage des amorces et préférentiellement par leur extrémité 5' ou soit par marquage interne du fragment amplifié.

Le premier type de marquage a l'inconvénient de présenter une activité spécifique faible et par conséquent, limite la sensibilité de la révélation ultérieure. Il est possible de fixer un phosphate radioactif (³²P) en 5' des amorces. On aura un (³²P) par amorce. Si on fixe une biotine ou un fluorochrome, il est possible d'avoir au plus 3 à 4 marqueurs par molécule d'amorce.

Si des nucléotides radioactifs sont incorporés dans l'amplicon, l'activité spécifique est certes plus importante, mais il faut manipuler davantage de radioactivité. La tendance actuelle est de remplacer les méthodes de marquage isotopiques par des marquages froids (fluorophore, digoxigénine, biotine).

Les fluorophores sont sensibles aux changements d'environnement : des variations dans les conditions expérimentales (pH, présence d'éléments oxydants, etc...) peuvent déplacer la longueur d'onde d'émission. De plus, les phénomènes d'extinction de fluorescence (ou quenching) ont largement été décrits. L'incorporation de nucléotides marqués par un fluorophore ou la digoxigénine ou la biotine par les polymérases est peu efficace car ces nucléotides présentent un fort encombrement stérique qui gène la réaction de polymérisation par PCR.

La demande de brevet PCT WO 98 11249 décrit une méthode de détection de protéines mutées consistant à associer à l'extrémité N-terminale des protéines synthétisées *in vitro* une séquence peptidique ou protéine rapporteur, fusionnée avec la protéine à détecter, susceptible d'être reconnue par un ligand pour purifier les dites protéines en vue de leur analyse.

Le marquage radioactif de protéines peut se faire en utilisant des acides aminés marqués par un isotope, ce qui implique la manipulation de radioactivité. Le marquage de protéines par une réaction antigène/anticorps peut pour sa part ne pas être assez sensible.

Le but de la présente invention est précisément d'offrir une méthode de détection de substances cibles sensible ne présentant pas les inconvénients ci-dessus.

Ce but est atteint grâce à une méthode de détection d'une substance cible dans un échantillon, caractérisée en ce qu'elle comprend les étapes suivantes :
a) le marquage spécifique de ladite substance, par un gène rapporteur et par les séquences nécessaires à l'expression dudit gène rapporteur *in vitro,*
b) la transcription et la traduction in vitro dans un système acellulaire dudit gène rapporteur, étant entendu que lorsque la substance cible est une séquence d'acide nucléique cible, la transcription et la traduction dudit gène rapporteur ne conduisent pas à une protéine rapporteur fusionnée avec une protéine codée par ladite séquence d'acide nucléique cible,
c) la détection *in vitro* de la protéine rapporteur codée par ledit gène rapporteur.

L'exposé des différentes formes de mise en oeuvre de l'invention décrites ci-après sont conformes aux procédés définis dans les revendications.

La méthode de l'invention est donc basée sur un marquage consistant à associer à la substance cible une molécule d'ADN constituant un gène rapporteur pouvant être exprimé *in vitro.* Le marquage consiste donc à associer à la substance cible, un gène rapporteur placé sous le contrôle des séquences nécessaires à son expression.

Les promoteurs de transcription *in vitro* pouvant être utilisés dans le cadre de l'invention peuvent correspondre notamment aux promoteurs des phages T7, SP6, Qβ ou λ.

A l'étape (b), on obtient la protéine codée par le gène rapporteur de façon à révéler spécifiquement la substance cible.

La révélation du marquage faisant l'objet du procédé de l'invention est sensible car elle met en oeuvre des étapes d'amplification lors des étapes de transcription (étape b), de traduction (étape b) et de détection (étape c). Cette amplification peut correspondre par exemple à un facteur 500 pour la transcription (Pokrovkaya et Gurevich, Analytical Biochemistry 220, 420-423 (1994).

La méthode de l'invention est aussi spécifique par le test de détection de la protéine à l'étape (c).

En outre la méthode de l'invention peut passer après l'étape de transcription par une étape d'amplification des transcrits par toutes techniques connues de l'homme de métier comme 3SR, NASBA (nucleic Acid Sequence-based Amplification), TMA (Transcription Mediated Amplification)

Dans le cas où la substance cible correspond à une molécule d'acide nucléique, l'amplification du signal de révélation peut commencer lors de l'étape (a) de la méthode de l'invention. Un jeu d'amorces ou de sondes particulières est utilisé de manière à amplifier spécifiquement une séquence et à associer à la présence d'une séquence oligonucléotidique spécifique un gène rapporteur pouvant être exprimé *in vitro.* Le gène rapporteur est exprimé uniquement si le gène cible est présent et amplifié. Comme indiqué ci-dessus, on entend par amplification du gène rapporteur ou du gène cible des réactions de type PCR (polymerase chain reaction), NASBA (nucleic acid sequence-based amplification), SDA (strand displacement amplification), bDNA (branched DNA signal amplification), par rolling circle, par des techniques dérivées de la PCR (nested PCR, multiplex PCR).

La méthode de l'invention est en outre rapide et reproductible, car toutes les réactions sont réalisées *in vitro*, ce qui permet de standardiser la détection. La méthode l'invention permet de réaaliser des détections qualitatives et quantitatives.

La méthode de l'invention est remarquable en ce qu'elle peut être appliquée à tout type de substance. Toutefois, l'invention s'applique plus particulièrement à des substances chimiques et biologiques, comme des anticorps, des fragments d'anticorps, des fragments nucléotidiques, des gènes, des récepteurs cellulaires, des peptides, des protéines, des acides aminés, des glycopeptides, des lipides, des glycolipides, des sucres, des polysaccharides, etc... . Dans une application particulière, la substance cible peut être le gène rapporteur lui-même.

On entend par substance cible marquée toute substance associée directement ou indirectement à un gène rapporteur pouvant être exprimée *in vitro.*

Le gène rapporteur est un gène qui pourra être transcrit et traduit *in vitro* en présence de séquences de régulation de son expression. La protéine pour laquelle code le gène rapporteur peut être détectée à l'étape (c) par toute technique connue de l'homme du métier. A titre d'exemple, le gène rapporteur peut être le gène de la protéine GFP (Green Fluorescent Protein) ou celui de la beta-lactamase (TEM-1). Dans le cas de la GFP, c'est l'émission de fluorescence qui est mesurée. Dans le cas de la beta-lactamase, c'est l'activité de cette enzyme qui est mesurée en incubant une fraction de la réaction de traduction dans un tampon contenant de la nitrocéphine. La nitrocéphine est une beta-lactamine chromogénique qui a la propriété de virer du jaune au rouge lorsqu'elle est hydrolysée par une beta-lactamase. Tout autre gène rapporteur peut être envisagé dans le procédé de l'invention, comme ceux de la beta-galactosidase, la beta-glucuronidase, la luciférase, la peroxydase ou une microperoxydase, etc... .

Le gène rapporteur code avantageusement pour une enzyme. La spécificité du marquage de la substance cible à l'étape (a) de la méthode de l'invention peut être réalisée par toute méthode directe ou indirecte connue de l'homme du métier.

Par méthode directe, on entend le fait que la substance cible est associée directement au gène et aux éléments nécessaires à l'expression dudit gène rapporteur *in vitro.* Il s'agit par exemple du cas décrit ci-après d'une molécule d'acide nucléique recombinante où la substance cible est une séquence nucléique comprise dans ladite molécule d'acide nucléique recombinante comprenant également le gène rapporteur et les séquences nécessaires à son expression *in vitro.*

Par méthode indirecte, on entend le fait que la substance cible est associée au gène rapporteur et aux séquences nécessaires à son expression *in vitro,* par l'intermédiaire d'un ligand spécifique de la substance cible. Ce ligand est associé au gène rapporteur et aux éléments nécessaires à son expression *in vitro.* C'est donc la mise en contact de ce ligand avec la substance cible qui permet de réaliser le marquage spécifique de la substance cible. Il s'agit par exemple d'un anticorps marqué par le gène rapporteur et les séquences nécessaires à son expression *in vitro* qui est capable de reconnaître spécifiquement une substance cible constituée d'un antigène. On entend par couple substance cible / ligand, par exemple : un antigène / un anticorps, une séquence nucléique / une séquence nucléique, une sonde, un récepteur / un ligand du récepteur, etc... .

Dans le mode de réalisation indirecte, la méthode de l'invention consiste à l'étape (a) à mettre en contact l'échantillon susceptible de contenir la substance cible avec un ligand spécifique de cette substance cible, ledit ligand étant marqué par un gène rapporteur et par les séquences nécessaires à l'expression dudit gène rapporteur *in vitro.* La suite de la méthode comprend comme précédemment les étapes (b) et (c).

Le marquage du ligand spécifique de la substance cible peut être comme précédemment un marquage direct ou indirect.

Selon la méthode indirecte de l'invention, l'association du gène rapporteur à une substance cible correspondant à une protéine admet plusieurs modes de réalisation. En effet, la fixation d'une molécule d'acide nucléique constituée du gène rapporteur et des séquences nécessaires à son expression *in vitro,* sur une protéine peut être réalisée par des techniques connues de l'homme du métier mettant en oeuvre des composés de liaison, comme :
- la streptavidine/biotine (Kipriyanov et al., (1995). Hum Antibodies Hybridomas 6 (3), 93-101)
- un peptide correspondant à la polylysine (Avrameas et al., (1998). PNAS 95 (10), 5601-6 ; Curiel et al., (1992). Hum Gene Ther 3 (2), 147-54 ; Wu et al., (1991). J Biol Chem 266 (22), 14338-42 ; Kwoh et al., (1999). Biochim Biophys Acta 1444 (2), 171-90 ; Wu et al., (1994). J Biol Chem 269 (15), 11542-6
- le p-azido-tetrafluoro-benzyl (Ciolina et al., (1999). Bioconjug Chem 10 (1), 49-55).
- Les triple hélices d'ADN (Neves et al., (1999). FEBS Lett 453 (1-2), 41-5 ).

La méthode de l'invention par marquage direct trouve plusieurs formes de mise en oeuvre qui seront présentées ci-après.

L'échantillon sur lequel est réalisée la méthode de l'invention peut-être tout prélèvement humain, animal, végétal, environnemental, ou toute matière susceptible d'être marquée par un gène rapporteur possédant toute information pour son expression *in vitro.*

Une forme préférée de mise en oeuvre du procédé de l'invention concerne la détection d'une séquence nucléique cible, avantageusement par la méthode directe.

La description ci-après concerne donc une méthode de détection *in vitro* d'une séquence nucléique dans un échantillon. Cette méthode comprend les étapes suivantes
a) la préparation, à partir de l'échantillon d'une molécule d'acide nucléique recombinante comprenant dans le sens 5' vers 3', un promoteur d'ARN polymérase, un gène rapporteur possédant les séquences nécessaires à son expression et éventuellement un terminateur d'ARN polymérase, la séquence d'acide nucléique cible étant localisée entre deux quelconques de ces éléments.
b) la transcription et la traduction des produits d'amplification obtenus à l'étape (a),
c) la révélation de l'activité de la protéine codée par le gène rapporteur, désignée la molécule rapporteur, obtenue à l'étape (b) par tout moyen approprié.

Différentes formes particulières de mise en oeuvre de la détection spécifique d'une substance cible correspondant à une séquence d'acide nucléique par marquage direct sont citées ci-après.

La méthode de l'invention est adaptée à la détection de séquences cibles pouvant correspondre à un gène, une fraction de gène ou à tout autre fragment d'acide nucléique.

La méthode de l'invention offre l'avantage de pouvoir détecter spécifiquement une séquence cible dans un échantillon à analyser et de travailler ultérieurement directement sur cette séquence cible.

La méthode de l'invention est aussi remarquable en ce qu'elle est tout particulièrement adaptée à la détection de séquences d'acide nucléique cibles codant pour un peptide ou une protéine n'ayant pas d'activité identifiable *in vitro.*

Selon une première forme préférée de réalisation de la méthode de l'invention la préparation de la molécule d'acide nucléique de l'étape (a) est réalisée par amplification *in vitro* de la séquence d'acide nucléique cible.

Il peut s'agir d'une amplification par PCR ou par des techniques dérivées de la PCR du type RT-PCR, nested PCR, multiplex PCR, ou des techniques différentes de la PCR : NASBA (nucleic acid sequence-based amplification) ou cercle roulant ou autres.

Dans une forme de réalisation de la méthode de l'invention, désignée ci-après "universelle", la première étape (a) de la méthode est basée sur la réalisation d'une réaction d'amplification de la séquence cible, si bien entendu celle-ci est présente dans l'échantillon analysé, à l'aide de deux amorces désignées sens et antisens définies ci-dessous:
- une amorce sens comprenant au moins une partie homologue à la région 5' de la séquence cible,
- une amorce antisens, comprenant au moins une partie homologue à la région 3' de la séquence cible,
lesdites amorces permettant après amplification de la séquence d'acide nucléique cible, et après l'étape (b) l'expression du gène rapporteur.

L'invention concerne donc aussi un jeu d'amorces susceptible d'être utilisé à l'étape (a) d'une méthode selon l'invention caractérisé en ce qu'il comprend :
- une amorce sens comprenant au moins une partie homologue à la région 5' de la séquence cible,
- une amorce antisens, comprenant au moins une partie homologue à la région 3' de la séquence cible,
lesdites amorces permettant après amplification de la séquence d'acide nucléique cible, et après l'étape (b) l'expression d'un gène rapporteur.

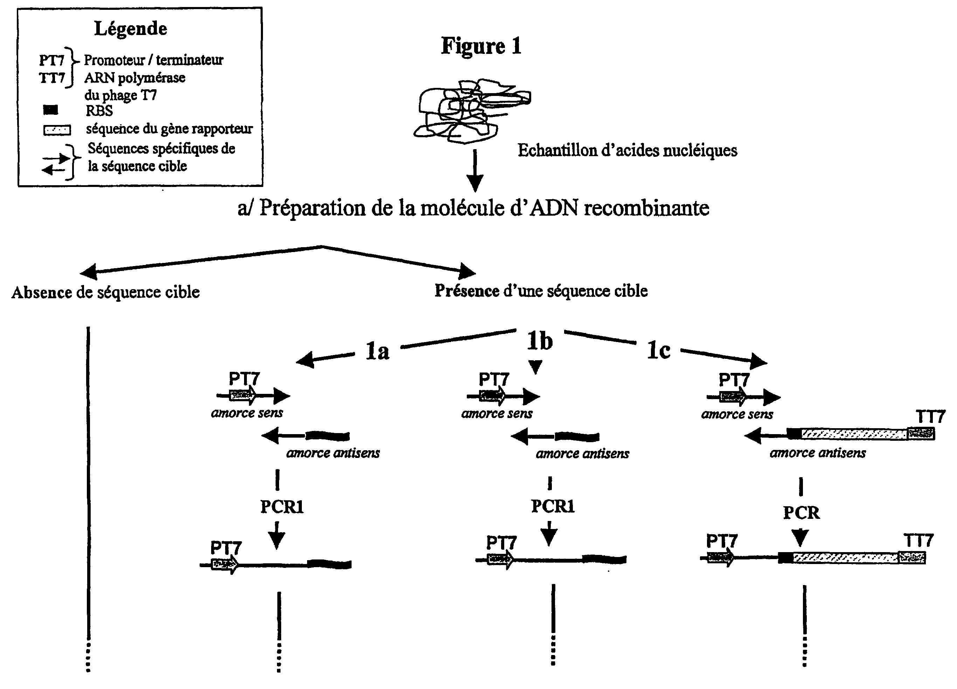

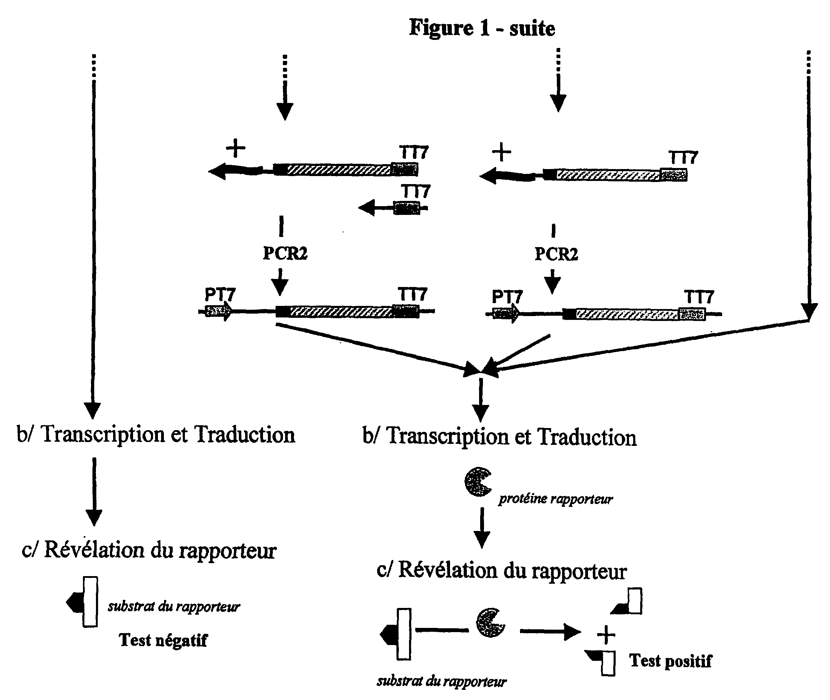

Dans un premier mode de mise en oeuvre particulier de la méthode universelle de l'invention, l'étape (a) comprend les trois réactions suivantes (figure 1a) :
a') l'amplification de la séquence cible avec une paire d'amorces dont l'amorce sens possède un promoteur d'ARN polymérase et l'amorce antisens possède une région 5' homologue au début de la séquence du gène rapporteur, et
a'') la mise en contact des produits d'amplification de la réaction précédente avec le gène rapporteur possédant éventuellement un terminateur d'ARN polymérase pour sa transcription, un site de fixation des ribosomes pour sa traduction, de façon à hybrider lesdits produits d'amplification avec ledit gène rapporteur, puis
a''') l'amplification des produits de l'étape (a'') avec une paire d'amorces, dont l'amorce sens est similaire à celle de l'étape (a') et l'amorce antisens comprend une partie homologue à une région en aval du gène rapporteur.

Dans ce mode de mise en oeuvre, le mélange de réaction PCR contient l'ADN échantillon, deux amorces sens et antisens, la séquence rapporteur et une troisième amorce homologue à une région en aval du gène rapporteur. La première amplification de l'étape (a') et la seconde amplification de l'étape (a''') peuvent être réalisées simultanément ou non.

Comme montré sur la figure la en annexe, les premiers cycles de la réaction PCR1 permettent l'amplification de la séquence cible. Une fois que cette séquence est présente en quantité suffisante dans le milieu réactionnel, elle sert de méga-amorce et vient s'hybrider sur la séquence rapporteur, qui est alors amplifiée grâce à la troisième amorce.

Dans un deuxième mode de mise en oeuvre de la méthode universelle de l'invention, l'étape (a) comprend les deux réactions suivantes (figure 1b) :
a') l'amplification de la séquence cible avec une paire d'amorces dont l'amorce sens possède un promoteur d'ARN polymérase et l'amorce antisens possède une région 5' homologue au début de la séquence du gène rapporteur, et
a'') l'amplification des produits de l'étape (a') avec une paire d'amorces dont l'amorce sens est identique à celle de l'étape (a') et l'amorce antisens est une méga-amorce constituée du gène rapporteur possédant éventuellement un terminateur d'ARN polymérase pour sa transcription, un site de fixation des ribosomes pour sa traduction.

Dans ce mode de mise en oeuvre, l'amorce antisens possède une région 5' homologue au début de la séquence rapporteur, laquelle possède, comme précédemment toutes les informations pour sa propre traduction. Comme indiqué à la figure 1b en annexe, le mélange de réaction PCR contient l'ADN échantillon, deux amorces sens et antisens et une méga-amorce correspondant à la séquence rapporteur. L'amorce antisens est présente en moins grande quantité que l'amorce sens. Les premiers cycles de la réaction PCR1 permettent l'amplification de la séquence cible. Une fois que cette séquence est présente en quantité suffisante dans le milieu réactionnel, elle est amplifiée (PCR2) à l'aide de l'amorce sens de la PCR1 et de la méga-amorce.

Dans un troisième mode de mise en oeuvre de la méthode universelle de l'invention (figure 1c), l'étape (a) comprend la réaction d'amplification de la séquence cible avec une paire d'amorces dont l'amorce sens possède un promoteur d'ARN polymérase et l'amorce antisens possède une région 5' comprenant une séquence codant pour un site de fixation des ribosomes , un gène rapporteur et éventuellement un terminateur de transcription.

Dans ce mode de mise en oeuvre représenté à la figure 1c en annexe, l'amorce antisens possède une région 3' spécifique de la séquence cible et une région 5' correspondant à une séquence codant pour un site de fixation des ribosomes , un gène rapporteur et éventuellement un terminateur de transcription. Le mélange de réaction PCR contient l'ADN échantillon, deux amorces sens et antisens.

L'invention concerne également un mélange de marquage par amplification pour la réalisation des modes de mises en oeuvre décrits ci-dessus. Un tel mélange comprend, les réactifs nécessaires à la réalisation des cycles d'amplification, et donc plus particulièrement, les quatre déoxynucléotides triphosphates, les sels et réactifs assurant l'activité optimale de l'ADN polymérase, ainsi que les différents types d'amorces décrits ci-dessus.

Un mélange de marquage par amplification tout particulièrement adapté à la réalisation de l'étape (a) du premier mode de mise en oeuvre de la méthode universelle selon l'invention comprend :
- une paire d'amorces dont l'amorce sens possède un promoteur d'ARN polymérase et l'amorce antisens possède une région 5' homologue au début de la séquence du gène rapporteur,
- le gène rapporteur possédant éventuellement un terminateur d'ARN polymérase pour sa transcription, et un site de fixation des ribosomes pour sa traduction, et
- une troisième amorce homologue à une région en aval du gène rapporteur.

Un mélange de marquage par amplification tout particulièrement adapté à la réalisation de l'étape (a) du deuxième mode de mise en oeuvre de la méthode universelle selon l'invention comprend :
- une paire d'amorces dont l'amorce sens possède un promoteur d'ARN polymérase et l'amorce antisens possède une région 5' homologue au début de la séquence du gène rapporteur, et
- une méga amorce constituée du gène rapporteur possédant éventuellement un terminateur d'ARN polymérase pour sa transcription et un site de fixation des ribosomes pour sa traduction.

Un mélange de marquage par amplification tout particulièrement adapté à la réalisation de l'étape (a) du troisième mode de mise en oeuvre de la méthode universelle selon l'invention comprend une paire d'amorces dont l'amorce sens possède un promoteur d'ARN polymérase et l'amorce antisens possède une région 5' comprenant une séquence codant pour un site de fixation des ribosomes , un gène rapporteur et éventuellement un terminateur de transcription.

L'invention a donc également pour objet un kit pour la détection d'une séquence cible d'acide nucléique dans un échantillon conformément à la méthode universelle décrite précédemment.

Un kit pour la détection d'une séquence cible d'acide nucléique dans un échantillon selon l'invention comprend un jeu d'amorces défini ci-dessus, un mélange nécessaire à l'amplification, les nucléotides triphosphates, une ARN polymérase ADN dépendante, une ADN polymérase ADN dépendante, un extrait cellulaire de traduction, les mélanges nécessaires à la transcription, à la traduction et éventuellement à la révélation de la molécule rapporteur, et éventuellement une ou plusieurs substances permettant de révéler l'activité de la molécule rapporteur, où la transcription et la traduction dudit gène rapporteur ne conduisent pas à une protéine rapporteur fusionnée avec une protéine codée par ladite séquence d'acide nucléique cible.

Un exemple particulier d'un kit selon l'invention comprend outre, l'un des mélanges d'amplification ci-dessus, une ADN polymérase ADN dépendante, les nucléotides triphosphates et les désoxynucléotides triphosphates, une ARN polymérase ADN dépendante, un extrait cellulaire de traduction, les mélanges nécessaires à l'amplification, à la transcription, à la traduction et éventuellement à la révélation de la molécule rapporteur, et éventuellement une ou plusieurs substances permettant de révéler l'activité de la molécule rapporteur.

La méthode de l'invention, au niveau de l'étape (a), peut également être mise en oeuvre sur la base des propriétés des sondes dites "Padlock (1, 2, 3, 4, WO 97/19193). Cette forme de mise en oeuvre est plus particulièrement adaptée à la détection de séquences cibles possèdant une mutation ponctuelle.

La détection de séquences cibles utilisant les sondes "Padlock" constitue donc une alternative aux modes de mise en oeuvre précédemment décrits de la méthode de l'invention dite universelle.

L'invention se rapporte donc également à une méthode de détection *in vitro* d'une séquence d'acide nucléique cible dans un échantillon caractérisée en ce que la préparation de la molécule d'acide nucléique de l'étape (a) est réalisée par hybridation et ligation d'une sonde de type Padlock avec la séquence cible si elle est présente dans l'échantillon.

Ladite sonde est composée en 3' et 5' de segments séparés par la séquence complémentaire d'un gène rapporteur qui possède les séquences complémentaires d'un promoteur et éventuellement d'un terminateur d'ARN polymérase pour sa transcription, et la séquence complémentaire d'un site de fixation des ribosomes pour sa traduction *in vitro,* les séquences desdits segments 3' et 5' de la sonde Padlock étant complémentaires de la séquence cible recherchée de manière à former avec celle-ci un hybride jointif, et l'extrémité 5' de la sonde possédant un groupe phosphate de façon à permettre la circularisation de la sonde sous l'action d'une ligase. On utilisera de préférence une ligase nick-sealing. Ainsi, en absence de séquence cible, la sonde ne peut pas être circularisée.

Ladite sonde peut être également composée en 5' et 3' de segments séparés de 5' en 3' par la séquence d'un promoteur d'ARN polymérase, d'un site de fixation des ribosomes et de la séquence d'un gène rapporteur avec éventuellement un terminateur d'ARN polymérase.

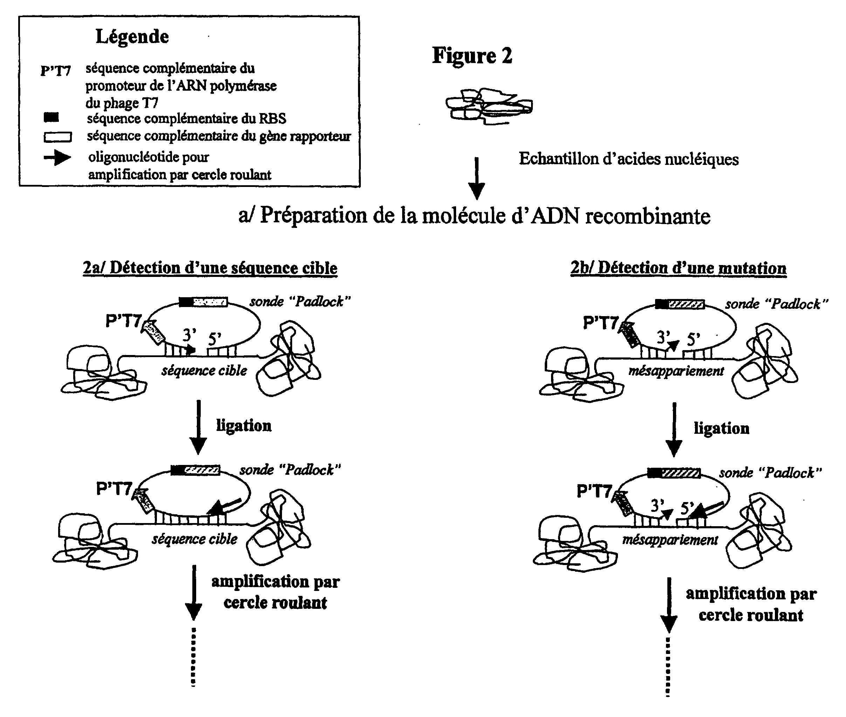

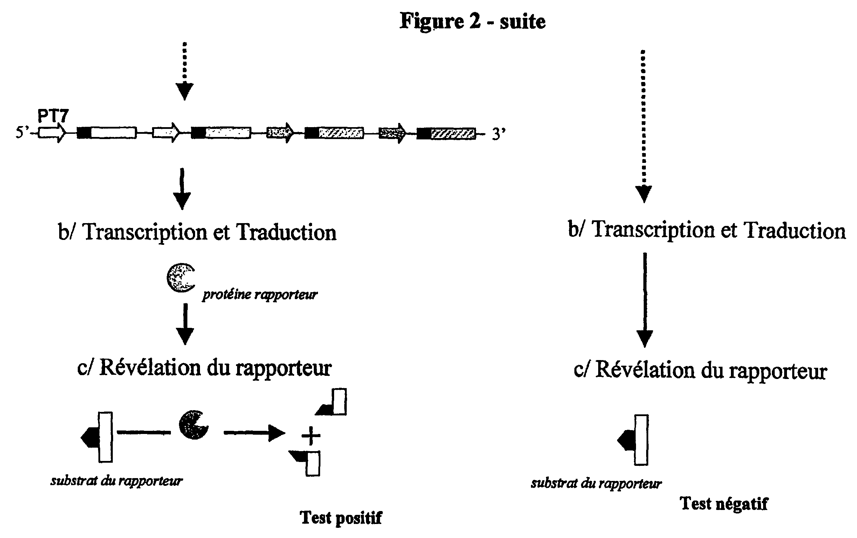

Les segments 3' et 5' de la sonde Padlock sont donc définis pour s'hybrider de manière complémentaire et jointive sur la séquence cible recherchée comme montré à la figure 2a en annexe.

L'invention concerne donc aussi une sonde Padlock susceptible d'être utilisée dans une méthode ci-dessus caractérisée en ce que les segments 3' et 5' de ladite sonde Padlock sont définis pour s'hybrider de manière complémentaire et jointive sur une séquence cible et où la transcription et la traduction du gène rapporteur ne conduisent pas à une protéine rapporteur fusionnée avec une protéine codée par ladite séguence d'acide nucléique cible.

Comme indiqué précédemment, la méthode de l'invention basée sur l'utilisation d'une sonde padlock peut être avantageusement utilisée pour la détection *in vitro* d'une séquence d'acide nucléique cible portant une mutation. Dans ce cas, la préparation de la molécule d'acide nucléique de l'étape (a) est réalisée par hybridation d'une sonde de type Padlock avec la séquence cible si elle est présente, ladite sonde correspondant à une des deux sondes décrites précédemment avec la particularité suivante : les séquences desdits segments 3' et 5' de la sonde Padlock sont complémentaires de la séquence cible recherchée de manière à former avec celle-ci un hybride où le nucléotide critique susceptible d'être muté, se trouve à leur point de jonction lorsqu'ils sont hybridés sur la séquence cible.

Ainsi, lorsque la méthode de l'invention est appliquée à la mise en évidence de la présence d'une mutation au niveau d'une séquence cible, comme montré sur la figure 2b, les segments 3' et 5' de la sonde "Padlock" sont définis de manière que le nucléotide critique, susceptible d'être muté, se trouve à leur point de jonction lorsqu'ils sont hybridés sur la séquence cible. En cas de mésappariement, la ligase ne pourra pas lier les deux extrémités et la circularisation de la sonde ne pourra pas se faire.

L'invention concerne donc aussi une sonde Padlock susceptible d'être utilisée dans la méthode ci-dessus, caractérisée en ce que les séquences desdits segments 3' et 5' de la sonde Padlock sont complémentaires d'une séquence cible portant un nucléotide susceptible d'être muté de manière à former avec celle-ci un hybride où ledit nucléotide se trouve à leur point de jonction lorsqu'ils sont hybridés sur la séquence cible.

Avantageusement, dans la méthode de l'invention basée sur l'utilisation d'une sonde padlock, après circularisation, la sonde est utilisée comme matrice pour sa réplication par cercle roulant. La réplication par cercle roulant est réalisée à l'aide d'une amorce complémentaire à la sonde padlock pour initier la réplication par une ADN polymérase, de façon à produire une matrice d'ADN possédant un enchaînement de gènes rapporteurs avec tous les signaux nécessaires pour son expression *in vitro* (soit, de 5' en 3', une répétition de l'élément suivant (promoteur d'ARN polymérase, de site de fixation des ribosomes , de gène rapporteur et éventuellement de terminateur d'ARN polymérase), ou le complémentaire de cet séquence selon la sonde choisie puis le brin complémentaire de cette matrice d'ADN est synthétisé à partir d'une deuxième amorce oligonucléotidique de façon à rendre cette matrice double brin.

Cette étape de préparation augmente la sensibilité de la détection grâce aux nombreuses copies du gène rapporteur produites à partir d'une seule molécule cible.

Une forme toute particulière de mise en oeuvre de la méthode de l'invention basée sur les sondes padlock consiste à effectuer à l'étape (b) directement la transcription du gène rapporteur sur la sonde Padlock après sa circularisation et la synthèse du brin complémentaire à l'aide d'une amorce complémentaire à la sonde padlock complémentaires de la cible.

Cette forme de mise en oeuvre comprend la préparation à l'étape (a) d'une sonde padlock correspondant à celles décrites précédemment permettant soit la détection d'une séquence d'acide nucléique, soit la détection d'une mutation sur une séquence d'acide nucléique. Cette sonde padlock est créée de façon que la transcription directe du gène rapporteur par l'ARN polymérase ne puisse avoir lieu que si la sonde padlock a été préalablement circularisée suite à son hybridation sur la séquence nucléique cible. Dans ce cas, il n'y a pas d'amplification par cercle roulant, mais uniquement la synthèse du brin complémentaire de la sonde padlock à l'aide d'une amorce complémentaire à la sonde padlock complémentaires de la cible, et éventuellement ligation puis transcription directe du gène rapporteur de la sonde padlock par l'ARN polymérase.

L'invention concerne également les kits pour la mise en oeuvre de la méthode de l'invention utilisant une sonde padlock.

Un tel kit est caractérisé en ce qu'il comprend une sonde comme décrit précédemment, une ADN polymérase ADN dépendante, une ligase nick-sealing, les nucléotides triphosphates et les désoxynucléotides triphosphates, une amorce pour initier la réplication, une amorce permettant la synthèse du deuxième brin d'ADN, une ARN polymérase ADN dépendante, les mélanges nécessaires à la ligation, à la réplication, à la transcription, à la traduction et à la révélation de la molécule rapporteur, où la transcription et la traduction du gène rapporteur ne conduisent pas à une protéine rapiporteur fusionnée avec une protéine codée par ladite séquence d'acide nucléique cible.

Un premier exemple de kit selon l'invention comprend plus particulièrement :
- Une des deux sondes de type Padlock décrites précédemment. Les séquences desdits segments 3' et 5' de la sonde Padlock sont complémentaires de la séquence cible recherchée de manière à former avec celle-ci un hybride jointif, et l'extrémité 5' de la sonde posséde un groupe phosphate de façon à permettre la circularisation de la sonde sous l'action d'une ligase.
- Une ADN polymérase ADN dépendante, une ligase nick-sealing, les nucléotides triphosphates et les désoxynucléotides triphosphates, une amorce pour initier la réplication en cercle roulant, une amorce permettant la synthèse du deuxième brin de la matrice d'ADN générée par cercle roulant, une ARN polymérase ADN dépendante, les mélanges nécessaires à la ligation, au marquage, à la éplication, à la transcription, à la traduction et à la révélation de la molécule rapporteur.

Un deuxième exemple de kit selon l'invention comprend plus particulièrement :
- une des deux sondes de type Padlock décrites précédemment. Les séquences desdits segments 3' et 5' de la sonde Padlock sont complémentaires de la séquence cible recherchée de manière à former avec celle-ci un hybride où le nucléotide critique susceptible d'être muté, se trouve à leur point de jonction lorsqu'ils sont hybridés sur la séquence cible, et l'extrémité 5' de la sonde posséde un groupe phosphate de façon à permettre la circularisation de la sonde sous l'action d'une ligase.
- Une ADN polymérase ADN dépendante, une ligase nick sealing, les désoxynucléotides triphosphates, les nucléotides triphosphates, une amorce pour initier la réplication en cercle roulant, une amorce permettant la synthèse du deuxième brin de la matrice d'ADN générée par cercle roulant, une ARN polymérase ADN dépendante, les mélanges nécessaires à la ligation, au marquage, à la réplication, à la transcription, à la traduction et à la révélation de la molécule rapporteur.

Un troisième type de kit pour la mise en oeuvre de la méthode toute particulière de l'invention ci-dessus utilisant une sonde padlock comprend :
- Une des sondes de type Padlock décrites précédemment permettant soit la détection d'une séquence d'acide nucléique, soit la détection d'une mutation sur une séquence d'acide nucléique.
- Une ADN polymérase ADN dépendante, une ligase, les désoxynucléotides triphosphates, les nucléotides triphosphates, une amorce pour synthétiser le brin complémentaire de la sonde padlock circularisée, une ARN polymérase ADN dépendante, les mélanges nécessaires à la ligation, au marquage, à la réplication de l'ADN, à la transcription, à la traduction et éventuellement à la révélation de la molécule rapporteur, et éventuellement une ou plusieurs substances permettant de révéler l'activité de la molécule rapporteur.

La méthode de l'invention peut également être mise en oeuvre dans le cadre d'une amplification isothermique aussi désignée CIA pour "Continuous Isothermic Amplification" du type décrit dans la demande de brevet internationale PCT No. WO96/01327.

Dans ce mode de réalisation de la méthode de l'invention, la séquence d'acide nucléique cible recherchée est isolée d'un échantillon d'acide nucléique à l'étape (a) par amplification isothermique spécifique à l'aide d'une ADN polymérase ADN dépendante et de deux amorces spécifiques de la séquence cible, dont l'une au moins est constituée d'une partie 3' pouvant s'hybrider spécifiquement à la séquence cible et d'une partie 5' comportant au moins une séquence répétée inversée pour former à une température adéquate une structure dite en "épingle à cheveux". La température de fusion de la structure double brin en épingle à cheveux est de préférence inférieure ou égale à la température de fusion de la partie de l'amorce s'hybridant spécifiquement avec la séquence cible. La différence entre les deux températures de fusion est par exemple de 10°C. En outre, l'une des amorces porte la séquence d'un promoteur de transcription d'une ARN polymérase ADN dépendante, comme le promoteur de l'ARN polymérase du phage T7 et l'autre amorce porte un gène rapporteur possédant en 5' un site de fixation des ribosomes.

Dans ce mode de réalisation de l'invention, on peut citer tout particulièrement comme gène rapporteur, un gène codant pour une microperoxydase.

Selon une forme de mise en oeuvre avantageuse, les deux amorces possèdent une séquence répétée inversée pour former à une température adéquate une structure en épingle à cheveux. Les séquences répétées inversées des deux amorces peuvent être identiques ou différentes. On préfère qu'elles soient différentes de façon à éviter une hybridation entre elles.

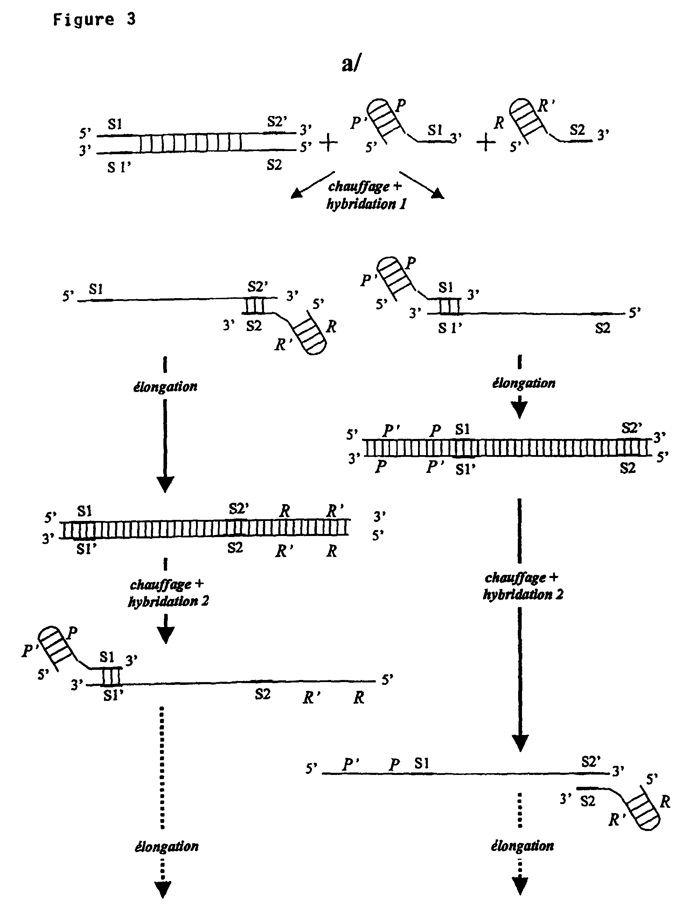

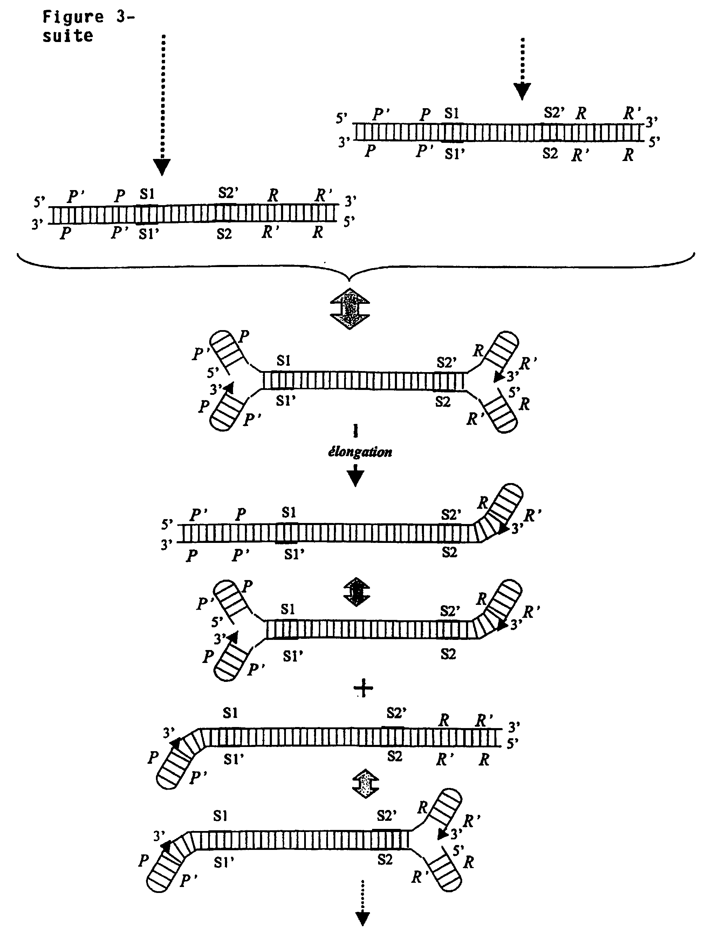

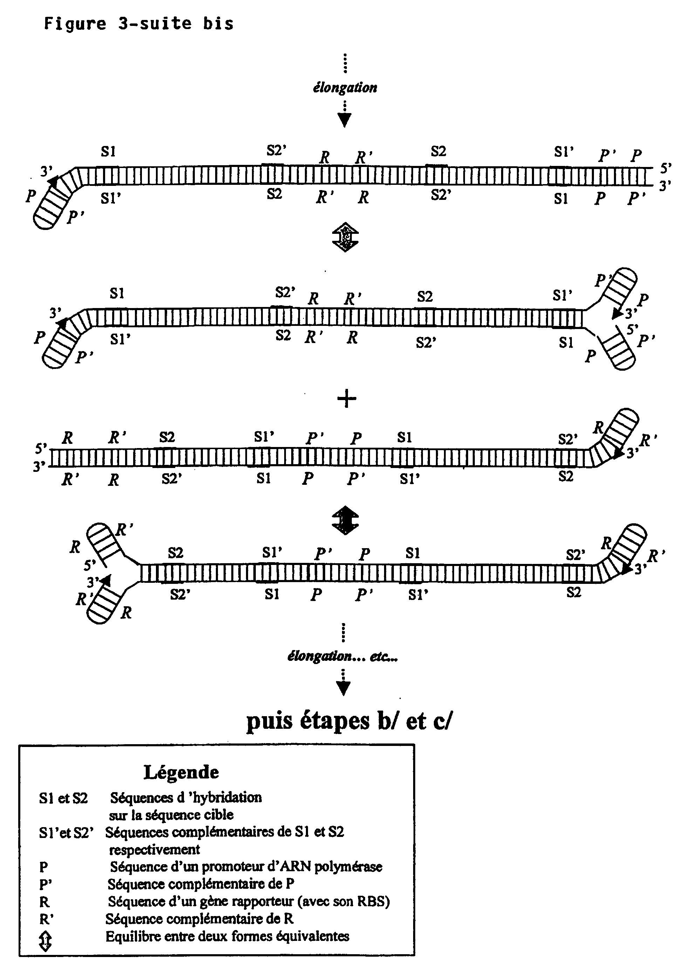

Une représentation schématique de la méthode de l'invention basée sur une amplification isothermique est donnée à la figure 3 en annexe. Parmi les différentes paires d'amorces pouvant être utilisées dans ce mode de réalisation de la méthode de l'invention, on peut citer plus particulièrement la paire d'amorces préférée, désignée A, suivante :
- une amorce sens constituée de 5' en 3', de la séquence complémentaire inversée d'un promoteur d'ARN polymérase, de la séquence dudit promoteur d'ARN polymérase, et d'une séquence spécifique capable de s'hybrider en amont de la séquence cible,
- une amorce antisens constituée de 5' en 3', d'un site site de fixation des ribosomes , d'un gène rapporteur, de la séquence complémentaire inversée dudit gène rapporteur et dudit site de fixation des ribosomes , et d'une séquence spécifique capable de s'hybrider en aval de la séquence cible, l'une au moins desdites amorces comprend éventuellement un site de restriction.

L'invention concerne aussi un jeu d'amorces susceptible d'être utilisé dans la méthode ci-dessus caractérisé en ce qu'il comprend :
- une amorce sens comprenant au moins une partie homologue à la région 5' de la séquence cible,
- une amorce antisens, comprenant au moins une partie homologue à la région 3' de la séquence cible,
lesdites amorces permettant après amplification de la séquence d'acide nucléique cible, et après l'étape (b) l'expression d'un gène rapporteur, et l'une au moins desdites amorces comportant une séquence inversée répétée en 5'.

L'amplification isothermique est réalisée avec le milieu réactionnel suivant :
- la molécule d'acide nucléique matrice possédant la séquence cible,
- les quatre déoxynucléotides triphosphates,
- des sels et réactifs assurant une activité optimale de l'ADN polymérase,
- une ADN polymérase ADN dépendante,
- une paire d'amorces spécifiques de la séquence cible à amplifier et comprenant les séquences inversées répétées définies ci-dessus.

L'ADN polymérase ADN dépendante peut être thermostable ou mésophile. Avantageusement, on utilise une ADN polymérase mésophile pourvue d'une activité de déplacement de brin, comme par exemple le fragment de Klenow de l'ADN polymérase I d'E. Coli. L'ajout de cette ADN polymérase ADN dépendante est effectué en début de réaction et après les deux premières étapes de chauffage nécessaires à la dénaturation de l'ADN préalablement à la mise en oeuvre de l'amplification isothermique.

L'étape (a) de la méthode de l'invention consiste : à chauffer le mélange réactionnel ci-dessus de manière à séparer les brins d'ADN, puis à refroidir pour permettre l'hybridation des amorces, et à se placer à la température adéquate pour l'élongation par l'ADN polymérase. Ces étapes de chauffage, d'hybridation et d'élongation sont répétées une deuxième fois avant d'obtenir une séquence cible simple brin dont les extrémités sont constituées de séquences répétées inversées. Cette molécule d'acide nucléique recombinante présente à la fois un rôle de matrice et d'amorce grâce à la structure en épingle à cheveux à chacune de ses extrémités. Contrairement à ce qu'on connaît avec la PCR, l'amplification se déroule ici de façon spontanée et à température constante. La température est choisie de manière à permettre :
- l'équilibre des séquences répétées inversées entre la forme linéaire et la forme en épingle à cheveux,
- l'élongation des amorces par l'ADN polymérase.

Comme montré sur la figure 3 en annexe, les produits d'amplification atteignent rapidement une taille importante, ce qui peut limiter l'élongation. Les amorces définissant la taille du fragment amplifié sont choisies pour optimiser l'amplification de la séquence cible associée à un gène rapporteur.

Un déroulement préféré de l'étape (a) d'amplification isothermique selon l'invention est le suivant :
i) on chauffe un mélange réactionnel comprenant l'échantillon d'acide nucléique dans lequel la séquence cible est éventuellement présente, les quatre déoxynucléotides triphosphates, des sels et réactifs assurant une activité optimale de l'ADN polymérase, une ADN polymérase ADN dépendante si celle-ci est thermophile, (sinon iii), une paire d'amorces spécifiques de la séquence cible à amplifier et comprenant les séquences inversées répétées, de manière à séparer les brins d'ADN, puis
ii) on refroidit pour permettre l'hybridation des amorces, et
iii) on ajoute de l'ADN polymérase si celle-ci est mésophile,
iv) on place le mélange réactionnel à la température adéquate pour l'élongation par l'ADN polymérase,
v) on répète une deuxième fois, les étapes de chauffage (i), d'hybridation (ii) d'ajout de l'ADN polymérase si elle est mésophile (iii), et d'élongation (iv) afin d'obtenir une séquence cible dont les extrémités sont constituées de séquences répétées inversées,
vi) on laisse la réaction d'amplification s'effectuer avec comme matrice et amorce les produits de l'étape précédente, à température constante choisie de manière à permettre :
   - l'équilibre des séquences répétées inversées entre la forme linéaire et la forme en épingle à cheveux,
   - l'élongation des amorces par l'ADN polymérase ADN dépendante.

En outre, les produits d'amplification peuvent être coupés spécifiquement par une enzyme de restriction, pendant ou après la réaction d'amplification de la séquence cible associée au gène rapporteur de l'étape (a). Le site de restriction se situe de préférence au niveau d'une des amorces et tout préférentiellement dans une boucle d'une épingle à cheveux, plus précisément dans deux séquences répétées inversées d'une des amorces.

Le choix des amorces et du site de coupure de l'enzyme sera défini pour une transcription efficace du gène rapporteur, de façon à ce que cette coupure n'altère pas la transcription ultérieure du gène rapporteur, où la transcription et la traduction du gène rapporteur ne conduisent pas à une protéine rapporteur fusionnée avec une protéine codée par ladite séquence d'acide nucléicrue cible.

Dans le cas de la paire d'amorces préférée A décrite précédemment, le site de coupure peut être situé sur les deux amorces et alors être identique ou différent sur chacune des amorces.

L'invention a donc également pour objet un jeu d'amorces comme défini ci-dessus susceptible d'être utilisé dans la méthode précédente caractérisé en ce que l'une au moins des deux amorces comporte un site de restriction. Si les deux possèdent un site de restriction, ceux-ci peuvent être identiques ou différents.

De préférence, le site de restriction présent sur l'une au moins des amorces se situe dans une boucle d'une épingle à cheveux, plus précisément entre deux séquences répétées inversées d'une des amorces.

L'invention concerne aussi le mélange réactionnel pour la réalisation de la méthode d'amplification isothermique ci-dessus comprenant les quatre déoxynucléotides triphosphates, des sels et réactifs assurant une activité optimale de l'ADN polymérase, une ADN polymérase ADN dépendante, une paire d'amorces spécifiques de la séquence cible à amplifier et comprenant en 5' les séquences inversées répétées contenant les séquences rapporteurs.

L'invention se rapporte encore à un kit pour la mise en oeuvre de la méthode de détection d'une séquence cible dans un échantillon d'ADN comprenant un jeu d'amorces comme défini ci-dessus, un mélange réactionnel d'amplification, éventuellement une ou plusieurs enzymes de restriction, une ARN polymérase ADN dépendante, une ADN polymérase ADN dépendante, les mélanges nécessaires, à la transcription, à la traduction et à la révélation de la molécule rapporteur, où la transcription et la traduction du gène rapporteur ne conduisent pas à une protéine rapporteur fusionnée avec une protéine codée par ladite séquence d'acide nucléique cible.

En dehors des exemples spécifiques de kit décrits précédemment où la substance cible est une séquence d'acide nucléique cible, l'invention concerne les kits pour la mise en oeuvre de la méthode de l'invention, quelque soit la substance cible un tel kit est caractérisé en ce qu'il comprend au moins un gène rapporteur, les mélanges nécessaires à la transcription, la traduction et à la révélation de la protéine codée par le gène rapporteur. Il est aussi possible de combiner dans un seul tube plusieurs détections selon la méthode de l'invention. Dans ce cas, des rapporteurs différents sont utilisés pour détecter chacune des substances cibles.

Il est encore possible d'utiliser le même rapporteur pour plusieurs substances cibles. Un résultat positif indique alors uniquement la présence de l'une ou l'autre des substances cibles.

La réaction de transcription et de traduction (étape b) peut être décomposée en deux étapes distinctes ou simultanées. Dans ce dernier cas, les réactions de transcription et de traduction sont réalisées simultanément. En revanche, la décomposition des étapes permet d'optimiser plus facilement les rendements de chaque étape, et ainsi de produire des quantités plus importantes de la protéine rapporteur, ce qui trouve toute son utilité dans le cas d'enzymes de faible activité spécifique.

Le découplage entre la transcription et la traduction permet également d'éviter les problèmes de dégradation de la matrice d'ADN par les nucléases si celle-ci a été préparée par PCR. En effet, les composants de la réaction de transcription sont peu contaminés par des nucléases, contrairement aux extraits de traduction.

En outre l'emploi d'extraits cellulaires de traduction différents selon l'origine du gène rapporteur permet d'optimiser la traduction. En effet, la phase de traduction du transcrit de l'étape (b) est avantageusement réalisée avec un extrait cellulaire de la même origine ou d'une origine proche de celle du gène rapporteur. On peut citer à titre d'exemple l'utilisation d'un extrait de traduction préparé à partir de cellules eucaryotes pour la traduction d'un gène rapporteur eucaryote. Dans un autre cas de figure, l'extrait de traduction est préparé à partir d'organismes extrêmophiles pour la traduction d'un gène rapporteur d'un même organisme ou d'un autre organisme extrêmophile de même type (thermophiles, halophiles, acidophiles, etc...).

Ces extraits spécifiques permettent d'améliorer l'efficacité de la traduction. Mais celle-ci peut aussi être réalisée avec un extrait standard comme par exemple un extrait *d'E. coli.*

Le procédé de l'invention est alors remarquable en ce qu'il met en oeuvre une adéquation entre la ponctuation d'expression des transcrits et les extraits de traduction utilisés. Ces extraits sont aussi caractérisés en ce que soit ils ne contiennent pas la propriété recherchée, soit ils la contiennent mais qu'elle n'est pas détectable dans les conditions de test réalisées pour détecter la fonction recherchée. Il s'agit par exemple de l'utilisation d'un extrait de traduction contenant une activité beta-galactosidase mésophile permettant de traduire un ARNm d'une beta-galactosidase thermophile et de la détection de l'activité de cette dernière à haute température, ce qui élimine l'activité beta-galactosidase mésophile.

Une mise en oeuvre particulière du procédé de l'invention consiste à utiliser à l'étape (b) un extrait de traduction qui soit en fait un mélange de plusieurs extraits de traduction. Il peut s'agir par exemple d'extrait de traduction de *E. coli* surexprimant une protéine chaperon A mélangé avec un extrait de traduction de *E. coli* surexprimant une protéine chaperon B. Tout type de mélange est envisageable du moment qu'il correspond aux caractéristiques décrites ci-dessus. De la même manière, il est possible d'utiliser un extrait de traduction dans lequel sont rajoutés un ou plusieurs tRNAs spécifiques d'un ou de plusieurs codons. Les extraits de traduction ainsi obtenus permettent alors de traduire des mRNA comportant ces codons spécifiques, comme par exemple la traduction d'un mRNA contenant un codon ambre en rajoutant dans l'extrait de traduction un tRNAs suppresseurs.

Le traitement de l'étape (b) avec un extrait de traduction peut aussi être réalisé avec un extrait standard de traduction quelle que soit l'origine de l'échantillon comme par exemple un extrait d'E. coli et/ou tout(s) autre(s) extrait(s) cellulaire(s) supplémenté(s) ou non par des molécules intéressantes comme celles, par exemple, indiquées précédemment (tRNA, chaperon...).

Il est également possible d'ajouter à l'extrait de traduction de l'étape (b) une ou plusieurs substances favorisant un repliement ou une maturation plus efficace des protéines exprimées, comme par exemple des chaperons, des détergents, des sulfobétaïnes, des extraits membranaires, etc... .

Selon une forme particulière de mise en oeuvre de l'étape (c), la révélation de l'activité de la protéine codée par le gène rapporteur, aussi désignée "molécule rapporteur" est réalisée par mise en contact de la molécule rapporteur avec un ou plusieurs substrats capables de révéler son activité.

Tout type de substrat spécifique peut être envisagé par l'homme de métier pour mettre en évidence la présence de l'activité de la protéine codée par le gène rapporteur. L'homme de métier pourra par exemple se référer à des ouvrages comme Methods In Enzymology ou Annual Review Of Biochemistry, dans lesquels un grand nombre de méthodes de dosage d'enzymes et de préparation de substrats ont été décrites.

La mesure de l'activité de la protéine de l'étape (c) peut être lue directement dans un lecteur de fluorimétrie si le rapporteur est par exemple la GFP ou de colorimétrie si le rapporteur est par exemple la beta-lactamase. Les lectures sont adaptées à la révélation du rapporteur. On peut également envisager des mesures par absorbance, par viscosité, par spectrophotométrie de masse etc... Il est tout a fait envisageable de réaliser une lecture en continu de l'activité du rapporteur, si ce dernier s'y prête.

Une forme particulière d'application du procédé de l'invention consiste à administrer la substance cible marquée par le gène rapporteur et les séquences nécessaires à son expression, par exemple à un organisme ou dans un processus, puis à rechercher par la poursuite des étapes de l'invention jusqu'à l'étape (c) de la méthode de l'invention, dans un échantillon prélevé sur ledit organisme ou dans ledit processus, la protéine codée par ledit gène rapporteur.

La méthode de l'invention peut être avantageusement automatisée notamment si le nombre d'échantillons à analyser est élevé. Les échantillons contenant les substances cibles sont alors placés sur un support pouvant correspondre à des biopuces ou des plaques de microtitration pouvant contenir plusieurs dizaines à plusieurs milliers d'emplacements. Ces supports sont placées sur un automate pour :
- la préparation des substances cibles (étape a),
- l'ajout des réactifs de transcription et de traduction (étape b). La révélation de la molécule rapporteur (étape c).

En conséquence, l'invention concerne un dispositif comprenant un agencement d'un ou plusieurs supports, de robots et d'un lecteur desdits supports pour la réalisation des étapes de 1a méthode décrite précédemment.

L'invention concerne également un procédé de marquage d'une substance correspondant à l'étape (a) de la méthode de l'invention décrite ci-dessus. L'invention concerne donc aussi une substance marquée par un gène rapporteur et les éléments nécessaires à l'expression in vitro dudit gène rapporteur susceptible d'être obtenue par ce procédé de marquage, où lorsque la substance cible est une séquence d'acide nucléique cible, la transcription et la traduction du gène rapporteur ne conduisent uas à une protéine rapporteur fusionnée avec une protéine codée par ladite séquence d'acide nucléique cible.

L'invention concerne enfin l'utilisation d'un gène rapporteur et des éléments nécessaires à l'expression in vitro dudit gène rapporteur comme marqueur d'une substance cible où lorsque la substance cible est une séquence d'acide nucléique cible, la transcription et la traduction du gène rapporteur ne conduisent pas à une protéine rapporteur fusionnée avec une protéine codée par ladite séquence d'acide nucléique cible.

D'autres avantages et caractéristiques de l'invention apparaîtront des exemples de réalisation de l'invention qui suivent.

### Exemple I : Marquage par PCR et détection.

1) Le gène codant pour la microperoxidase 8 (MP8) a été cloné dans le vecteur d'expression pET26b+.
Pour cela, deux oligonucléotides partiellement complémentaires MICRO1 et MICRO2 ont été hybridés produisant ainsi un fragment d'ADN double brin avec des extrémités protubérantes compatibles respectivement avec un site de restriction NdeI du côté de l'ATG du gène et avec un site XhoI à l'autre extrémité. Ce fragment d'ADN a été inséré dans le vecteur pET26b+ digéré par NdeI et XhoI. Ainsi, le plasmide obtenu contient le gène codant pour la MP8 sous contrôle du promoteur de la T7 ARN polymérase et de son terminateur situés de part et d'autre.
- Séquence MICRO1 : 5'TATGTGCGCACAATGTCATACAGTAGAATAATAAC
- Séquence MICRO2 : 5'TCGAGTTATTATTCTACTGTATGACATTGTGCGCACA
- Fragment d'ADN double brin :

Compatible                                    Compatible
  NdeI                                            XhoI
  ----                                           ------
5'TATG TGC GCA CAA TGT CAT ACA GTA GAA TAA TAA C
  3'AC ACG CGT GTT ACA GTA TGT CAT CTT ATT ATT GAGCT
  Met Cys  Ala Gln Cys His Thr Val Glu Stop Stop

2) Ce plasmide a ensuite servi de matrice lors de la PCR suivante :
plasmide : environ 100ng
amorce pET5' : 10pmol
amorce pET3' : 10pmol
Taqpol (Appligène) : 2U
DNTP : 200µM chaque
mix Taqpol Appligène : 1X

pET5'AGATCTCGATCCCGCGAAATTAATACG

pET3' CAAAAAACCCCTCAAGACCCGTTTAG

Les cycles d'amplification réalisés sont les suivants : 3mn à 94°, (30s à 94°, 30s à 60°, 1mn à 72°) 30 fois, 3mn à 72°
Cette PCR permet d'amplifier un fragment contenant, de 5' en 3', le promoteur de la T7 ARN polymérase, le site de fixation des ribosomes, le gène MP8 et le terminateur de transcription de la T7 ARN polymérase. Le produit PCR a été purifié par extraction phénol chloroforme et précipité à l'éthanol.
3) Le produit de la réaction PCR a été transcrit dans les conditions suivantes :
produit PCR : 100µg
T7ARN polymérase (NEB) : 300U
mix T7ARN polymérase (NEB) : 1X
MgCl₂ : 20mM
DTT : 20mM
NTP : 2mM chaque
3h à 37°

4) Des traductions ont été réalisées à partir de 0, 2.75 et 11µg d'ARN dans les conditions suivantes :
43.8µl d'extrait de traduction
0, 2.75 et 11µg d'ARN (0, 2.75, 11µg d'ARN)
Eau qsp 60µl
3h à 37°

5) Le dosage d'activité de la microenzyme a été réalisé selon le protocole suivant décrit par Hirayama et al. Deux gammes étalons ont été réalisées : une en présence de 10µl de traduction témoin (sans ARN) pour les points où on dose 10µl de traduction. Les résultats obtenus sont rapportés dans le tableau 1 ci-dessous, exprimés en unité de luminescence.

**Tableau 1**

| échantillon | 10µl de traduction |
|---|---|
| 0 (témoin) | 1760 |
| 0.1ng MP8 purifiée | 2368 |
| 1ng MP8 purifiée | 5248 |
| 10ng MP8 purifiée | 49088 |
| 100ng MP8 purifiée | 1530144 |
| traduction avec 2.75µg d'ARN | 2784 |
| traduction avec 11µg d'ARN | 6752 |

La gamme étalon obtenue permet d'estimer la quantité de MP8 produite à 0,14ng avec une traduction de 2,75µg dmontre que ce produit PCR peut être mis en évidence par la méthode objet de l'invention.

### Exemple II : Détection d'une cible d'ADN par padlock, et rolling circle.

1) L'oligonucléotide suivant a été préparé : 
5'P.TTCAGCAGGATTCCCCACAGCAAAATTATTTCTAGAGGGAAACCGTTGTGGTCTCC
homologue à la cible    Brin complémentaire promoteur T7
CTATAGTGAGTCGTATTAATTTTTATTATTCTACTGTATGACATTGTGCGCACATATGTA
brin complémentaire du gène MP8
TATCTCCTTCTTAAAGTTAAACCCGTCGATCATAAGGCTTGG 3'
Brin
complémentaire
du rbs
homologue à la cible
2) Le plasmide utilisé comme cible contient la séquence suivante : CTGTGGGGAATCCTGCTGAACCAAGCCTTATGATCGACGG
Cette séquence est reconnue par la sonde padlock ci-dessus.
3) La réaction de ligation a été réalisée comme suit :

| | |
|---|---|
| plasmide cible linéarisé | 2µg |
| mix Ampligase (Epicentre) | 1X |
| ampligase | 10U |
| oligo padlock | 0,2pmol |
| eau qsp 50µl | |

Les cycles de températures suivants ont ensuite été réalisés : 3mn à 94°, (10s à 94°, 10s à 55°, 1mn30 à 65°) 30 fois.
4) L'amplification en cercle roulant a été faite selon le protocole suivant :

| | |
|---|---|
| réaction de ligation | 10µl |
| oligo PADRCMP8 | 10pmol |
| dNTP | 384µM |
| Klenow polymerase | 5U |
| Eau qsp 25µl | |
| 2h à 37° | |

L'oligonucléotide PADRCMP8 a la séquence suivante : TTTAACTTTAAGAAGGAGATATAC. Il est complémentaire d'une partie de la sonde padlock et sert donc d'amorce au cercle roulant.
5) Synthèse du brin complémentaire.
50pmol d'oligonucléotide PADPCR5' a été rajouté ainsi que 5nmol de dNTP. Cet oligonucléotide est complémentaire du brin amplifié par cercle roulant. Le mélange réactionnel a été chauffé 5mn à 100°C puis laissé refroidir pour permettre l'hybridation de l'oligonucléotide sur le cercle roulant.
5U de Klenow DNA polymérase et 5U d'enzyme de restriction AseI ont ensuite été rajoutés. Après incubation 2h à 37°, on obtient des molécules d'ADN double brin. Un site AseI étant présent juste en amont de la séquence promotrice, la coupure par AseI permet de raccourcir les multimères et ainsi favorisera la transcription.
L'oligonucléotide PADPCR5' a la séquence suivante : 5'TTCAGCAGGATTCCCCACAG
6) Transcription.
La transcription du produit d'amplification a été réalisée dans les conditions suivantes :
produit de la réaction ci-dessus : 10µl
T7ARN polymérase (NEB) : 150U
mix T7ARN polymérase (NEB) : 1X
MgCl₂ : 20mM
DTT : 20mM
NTP : 2mM chaque
12h à 37°

7) Traduction : La traduction a été réalisée comme décrit dans l'exemple précédent.
8) Dosage : Le dosage d'activité de la microenzyme a été réalisé sur 10µl de traduction selon le protocole suivant décrit par Hirayama et al. (Hirayama O et al, 1997, Analytical Biochem, 247, p237-241) : le témoin négatif est une expérience complète où le plasmide cible a été remplacé par de l'eau à l'étape 3. La traduction et le dosage ont été fait deux fois avec des mix réactionnels différents.

Les résultats obtenus sont rapportés dans le tableau 2 ci-dessous exprimés en unité de luminescence.

**Tableau 2**

| échantillon | expérience 1 | expérience 2 |
|---|---|---|
| témoin | 280213 | 179333 |
| positif | 396000 | 268207 |

### RÉFÉRENCES BIBLIOGRAPHIQUES

1) Landegren, U., Samiotaki, M., Nilsson, M., Malmgren, H. et Kwiatkowski, M. (1996). Detecting genes with ligases. METHODS : A Compagnion to Methods in Enzymology 9, 84-90.
2) Landegren, U. et Nilsson M. (1997). Locked on target : strategies for future gene diagnostics. Ann. Med. 29, 585-590.
3) Nilsson M., Malmgren, H., Samiotaki, Kwiatkowski, M., Chowdhary, B.P. et Landegren, U (1994). Padlock Probes : Circularizing oligonucleotides for localized DNA detection. Science 265, 2085-2088.
4) Nilsson M. , Krejc, K., Koch, J., Kwiatkowski, M., Gustavsson, P.et Landegren, U. (1997). Padlock probes reveal single-nucleotide differences, parent of origin and *in situ* distribution of centromeric sequences in human chromosomes 13 and 21. Nature Genetics 16, 252-255.

## Revendications

1. Méthode de détection d'une substance cible dans un échantillon, **caractérisée en ce qu'**elle comprend les étapes suivantes :
a) le marquage spécifique de ladite substance par un gène rapporteur et par les séquences nécessaires à l'expression dudit gène rapporteur *in vitro,*
b) la transcription et la traduction in vitro dans un système acellulaire dudit gène rapporteur, étant entendu que lorsque la substance cible est une séquence d'acide nucléique cible, la transcription et la traduction dudit gène rapporteur ne conduisent pas à une protéine rapporteur fusionnée avec une protéine codée par ladite séquence d'acide nucléique cible,
c) la détection in vitro de la protéine rapporteur codée par ledit gène rapporteur.

2. Méthode de détection d'une substance cible dans un échantillon selon la revendication 1, **caractérisée en ce que** le marquage de l'étape (a) consiste à obtenir ladite substance marquée par un gène rapporteur muni d'un promoteur d'ARN polymérase et éventuellement d'un terminateur d'ARN polymérase pour sa transcription et un site de fixation des ribosomes pour sa traduction, de façon à ce qu'à l'étape (b), lorsque la substance cible est une séquence d'acide nucléique cible, la transcription et la traduction in vitro dans un système acellulaire dudit gène rapporteur, ne conduisent pas à une protéine rapporteur fusionnée avec une protéine codée par ladite séquence d'acide nucléique cible.

3. Méthode de détection d'une substance cible dans un échantillon selon l'une quelconque des revendications 1 à 2, **caractérisée en ce qu'**à l'étape (b) on procède à une étape d'amplification des transcrits avant la traduction.

4. Méthode de détection d'une substance cible dans un échantillon selon l'une quelconque des revendications 1 à 3, **caractérisée en ce qu'**à l'étape (a) la substance cible est associée directement au gène rapporteur et aux éléments nécessaires à l'expression dudit gène rapporteur *in vitro.*

5. Méthode de détection d'une substance cible dans un échantillon selon l'une quelconque des revendications 1 à 3, **caractérisée en ce qu'**à l'étape (a) la substance cible est associée au gène rapporteur et aux séquences nécessaires à son expression *in vitro,* par l'intermédiaire d'un ligand spécifique de la substance cible, ledit ligand étant marqué par un gène rapporteur et par les séquences nécessaires à l'expression dudit gène rapporteur *in vitro.*

6. Méthode de détection d'une séquence d'acide nucléiques cible dans un échantillon selon l'une quelconque des revendications 1 à 5, caractérisée en ce quelle comprend les étapes suivantes :
a) la préparation, à partir de l'échantillon d'une molécule d'acide nucléique recombinante comprenant dans le sens 5' vers 3', un promoteur d'ARN polymérase, un gène rapporteur possédant les séquences nécessaires à son expression et éventuellement un terminateur d'ARN polymérase, la séquence d'acide nucléique cible étant localisée entre deux quelconques de ces éléments,
b) la transcription et la traduction de la molécule d'acide nucléique recombinante obtenue à l'étape (a), la transcription et la traduction in vitro de ladite molécule d'acide nucléique recombinante ne conduisant pas à une protéine rapporteur fusionnée avec une protéine codée par ladite séquence d'acide nucléique cible,
c) la révélation de l'activité de la protéine codée par le gène rapporteur, désignée la molécule rapporteur, obtenue à l'étape (b) par tout moyen approprié.

7. Méthode de détection d'une séquence d'acide nucléique cible dans un échantillon selon la revendication 6, **caractérisée en ce que** la préparation de la molécule d'acide nucléique de l'étape (a) est réalisée par amplification *in vitro* de la séquence d'acide nucléique cible.

8. Méthode de détection d'une séquence d'acide nucléique cible dans un échantillon selon la revendication 7, **caractérisée en ce que** la préparation de la molécule d'acide nucléique de l'étape (a) est réalisée par amplification à l'aide de deux amorces :
- une amorce sens comprenant au moins une partie homologue à la région 5' de la séquence cible,
- une amorce antisens, comprenant au moins une partie homologue à la région 3' de la séquence cible,
lesdites amorces permettant après amplification de la séquence d'acide nucléique cible, et après l'étape (b) l'expression du gène rapporteur pour obtenir une protéine rapporteur non fusionnée avec une protéine codée par ladite séquence d'acide nucléique cible.

9. Méthode de détection d'une séquence d'acide nucléique cible dans un échantillon selon l'une des revendications 7 ou 8, **caractérisée en ce que** l'étape (a) comprend les trois réactions suivantes :
a') l'amplification de la séquence cible avec une paire d'amorces dont l'amorce sens possède un promoteur d'ARN polymérase et l'amorce antisens possède une région 5' homologue au début de la séquence du gène rapporteur, et
a'') la mise en contact des produits d'amplification de la réaction précédente avec le gène rapporteur possédant éventuellement un terminateur d'ARN polymérase pour sa transcription, un site de fixation des ribosomes pour sa traduction, de façon à hybrider lesdits produits d'amplification avec ledit gène rapporteur, puis
a''') l'amplification des produits de l'étape (a'') avec une paire d'amorces, dont l'amorce sens est similaire à celle de l'étape (a') et l'amorce antisens comprend une partie homologue à une région en aval du gène rapporteur.

10. Méthode de détection d'une séquence d'acide nucléique cible dans un échantillon selon l'une des revendications 7 ou 8, **caractérisée en ce que** l'étape (a) comprend les deux réactions suivantes :
a') l'amplification de la séquence cible avec une paire d'amorces dont l'amorce sens possède un promoteur d'ARN polymérase et l'amorce antisens possède une région 5' homologue au début de la séquence du gène rapporteur, et
a'') l'amplification des produits de l'étape (a') avec une paire d'amorces dont l'amorce sens est identique à celle de l'étape (a') et l'amorce antisens est une méga amorce constituée du gène rapporteur possédant éventuellement un terminateur d'ARN polymérase pour sa transcription, un site de fixation des ribosomes pour sa traduction.

11. Méthode de détection d'une séquence d'acide nucléique cible dans un échantillon selon l'une des revendications 7 ou 8, **caractérisée en ce que** l'étape (a) comprend la réaction d'amplification de la séquence cible avec une paire d'amorces dont l'amorce sens possède un promoteur d'ARN polymérase et l'amorce antisens possède une région 5' comprenant une séquence codant pour un site de fixation des ribosomes, un gène rapporteur et éventuellement un terminateur de transcription.

12. Méthode de détection *in vitro* d'une séquence d'acide nucléique cible dans un échantillon selon la revendication 6, **caractérisée en ce que** la préparation de la molécule d'acide nucléique de l'étape (a) est réalisée par hybridation et ligation d'une sonde de type Padlock avec la séquence d'acide nucléique cible.

13. Méthode de détection in vitro d'une séquence d'acide nucléique cible dans un échantillon selon la revendication 12, **caractérisée en ce que** ladite sonde Padlock est composée en 3' et 5' de segments séparés par la séquence complémentaire d'un gène rapporteur qui possède les séquences complémentaires d'un promoteur et éventuellement d'un terminateur d'ARN polymérase pour sa transcription, et la séquence complémentaire d'un site de fixation des ribosomes pour sa traduction *in vitro.*

14. Méthode de détection *in vitro* d'une séquence d'acide nucléique cible dans un échantillon selon la revendication 12, **caractérisée en ce que** ladite sonde est composée en 5' et 3' de segments séparés de 5' en 3' par la séquence d'un promoteur d'ARN polymérase, d'un site de fixation des ribosomes et de la séquence d'un gène rapporteur avec éventuellement un terminateur d'ARN polymérase.

15. Une sonde Padlock susceptible d'être utilisée dans une méthode selon l'une des revendications 12 à 14, **caractérisée en ce que** ladite sonde est composée en 5' et 3' de segments complémentaires de la séquence d'acide nucléique cible de manière à former avec celle-ci un hybride jointif, lesdits segments étant séparés par la séquence complémentaire d'un gène rapporteur qui possède les séquences complémentaires d'un promoteur et éventuellement d'un terminateur d'ARN polymérase pour sa transcription, et la séquence complémentaire d'un site de fixation des ribosomes pour sa traduction in vitro de façon à ce que la transcription et la traduction in vitro dans un système acellulaire dudit gène rapporteur ne conduisent pas à une protéine rapporteur fusionnée avec une protéine codée par ladite séquence d'acide nucléique cible.

16. Une sonde Padlock susceptible d'être utilisée dans une méthode selon l'une des revendications 12 à 14 **caractérisée en ce que** ladite sonde est composée en 5' et 3' de segments complémentaires de la séquence d'acide nucléique cible de manière à former avec celle-ci un hybride jointif, lesdits segments étant séparés par la séquence d'un promoteur d'ARN polymérase, d'un site de fixation des ribosomes et de la séquence d'un gène rapporteur avec éventuellement un terminateur d'ARN polymérase pour sa traduction in vitro de façon à ce que la transcription et la traduction in vitro dans un système acellulaire dudit gène rapporteur ne conduisent à pas une protéine rapporteur fusionnée avec une protéine codée par ladite séquence d'acide nucléique cible.

17. Une sonde Padlock selon l'une des revendications 15 ou 16, **caractérisée en ce que** les segments sont complémentaires d'une séquence d'acide nucléique cible portant un nucléotide muté de manière à former un hybride où ledit nucléotide muté se trouve au point de jonction desdits segments hybridés sur la séquence d'acide nucléique cible.

18. Méthode de détection *in vitro* d'une séquence d'acide nucléique cible dans un échantillon selon l'une des revendications 12 à 14, **caractérisée en ce que** après circularisation, la sonde est utilisée comme matrice pour sa réplication par cercle roulant.

19. Méthode de détection in vitro d'une séquence d'acide nucléique cible dans un échantillon selon la revendication 18, **caractérisée en ce que** la réplication par cercle roulant est réalisée à l'aide d'une amorce complémentaire à la sonde padlock complémentaires de la cible pour initier la réplication par une ADN polymérase, de façon à produire une matrice d'ADN possédant un enchaînement de gènes rapporteurs avec tous les signaux nécessaires pour son expression *in vitro,* et **en ce que** le brin complémentaire de cette matrice d'ADN est synthétisé à partir d'une deuxième amorce oligonucléotidique de façon à rendre cette matrice double brin.

20. Méthode de détection in vitro d'une séquence d'acide nucléique cible dans un échantillon selon l'une des revendications 12 à 14 ou 18, **caractérisée en ce que** l'on réalise à l'étape (b) directement la transcription du gène rapporteur sur la sonde Padlock après sa circularisation et la synthèse du brin complémentaire à l'aide d'une amorce complémentaire à la sonde padlock complémentaires de la cible.

21. Méthode de détection *in vitro* d'une séquence d'acide nucléique cible dans un échantillon selon l'une des revendications 6 à 8, **caractérisée en ce que** la séquence d'acide nucléique cible est isolée d'un échantillon d'acide nucléique à l'étape (a) par amplification isothermique spécifique à l'aide d'une ADN polymérase ADN dépendante et de deux amorces spécifiques de la séquence cible, dont l'une au moins est constituée d'une partie 3' pouvant s'hybrider spécifiquement à la séquence cible et d'une partie 5' comportant au moins une séquence répétée inversée pour former à une température adéquate une structure dite en "épingle à cheveux".

22. Méthode de détection *in vitro* d'une séquence d'acide nucléique cible dans un échantillon selon la revendication 21, **caractérisée en ce que** les deux amorces possèdent une séquence répétée inversée pour former à une température adéquate une structure en épingle à cheveux, lesdites séquences répétées inversées des deux amorces pouvant être identiques où différentes.

23. Méthode de détection *in vitro* d'une séquence d'acide nucléique cible dans un échantillon selon l'une des revendications 21 ou 22, **caractérisée en ce que** l'étape (a) d'amplification isothermique comprend les étapes suivantes :
i) on chauffe un mélange réactionnel comprenant l'échantillon d'acide nucléique dans lequel la séquence cible est éventuellement présente, les quatre déoxynucléotides triphosphates, des sels et réactifs assurant une activité optimale de l'ADN polymérase, une ADN polymérase ADN dépendante si celle-ci est thermophile, (sinon iii), une paire d'amorces spécifiques de la séquence cible à amplifier et comprenant les séquences inversées répétées, de manière à séparer les brins d'ADN, puis
ii) on refroidit pour permettre l'hybridation des amorces, et
iii) on ajoute de l'ADN polymérase si celle-ci est mésophile, puis
iv) on place le mélange réactionnel à la température adéquate pour l'élongation par l'ADN polymérase,
v) on répète une deuxième fois, les étapes de chauffage (i), d'hybridation (ii) d'ajout de l'ADN polymérase si elle est mésophile (iii), et d'élongation (iv) afin d'obtenir une séquence cible dont les extrémités sont constituées de séquences répétées inversées,
vi) on laisse la réaction d'amplification s'effectuer avec comme matrice et amorce les produits de l'étape précédente, à température constante choisie de manière à permettre :
- l'équilibre des séquences répétées inversées entre la forme linéaire et la forme en épingle à cheveux,
- l'élongation des amorces par l'ADN polymérase ADN dépendante.

24. Un jeu d'amorces pour l'amplification d'une séquence d'acide nucléique cible, susceptible d'être utilisé dans la méthode selon l'une des revendications 7 à 11 et 21 à 23, **caractérisé en ce qu'**il comprend :
- une amorce sens comprenant au moins une partie homologue à la région 5' de la séquence cible,
- une amorce antisens, comprenant au moins une partie homologue à la région 3' de la séquence cible,
lesdites amorces étant telles que :
- après amplification de la séquence d'acide nucléique cible, on obtient une molécule d'acide nucléique recombinante comprenant dans le sens 5' vers 3' un promoteur d'ARN polymérase, un gène rapporteur possédant les séquences nécessaires à son expression in vitro et éventuellement un terminateur d'ARN polymérase, et
- la transcription et la traduction dudit gène rapporteur *in vitro* dans un système acellulaire ne conduisent pas à une protéine rapporteur fusionnée avec une protéine codée par ladite séquence d'acide nucléique cible.

25. Un jeu d'amorce selon la revendication 24, **caractérisé en ce que** l'une au moins desdites amorces comporte une séquence inversée répétée en 5'.

26. Méthode de détection *in vitro* d'une séquence d'acide nucléique cible dans un échantillon selon l'une des revendications 21 à 23, **caractérisée en ce que** les produits d'amplification peuvent être coupés spécifiquement par une enzyme de restriction, pendant ou après la réaction d'amplification de la séquence cible.

27. Un jeu d'amorces selon la revendication 25, susceptible d'être utilisé dans la méthode selon la revendication 26, **caractérisé en ce que** l'une au moins des deux amorces comporte un site de restriction, et si les deux possèdent un site de restriction, ceux-ci peuvent être identiques ou différents.

28. Un jeu d'amorces selon la revendication 27, **caractérisé en ce que** le site de restriction présent sur l'une au moins des amorces se situe dans une boucle d'une épingle à cheveux, plus précisément entre deux séquences répétées inversées d'une des amorces.

29. Utilisation d'un gène rapporteur et des éléments nécessaires à son expression *in vitro* dans un système acellulaire pour le marquage spécifique d'une substance cible contenu dans un échantillon, ledit marquage étant tel que lorsque la substance cible est une séquence d'acide nucléique cible, la transcription et la traduction dudit gène rapporteur ne conduisent pas à une protéine rapporteur fusionnée avec une protéine codée par ladite séquence d'acide nucléique cible.

30. Un kit pour la mise en oeuvre de la méthode de détection d'une substance cible selon l'une des revendications 1 à 6, **caractérisé en ce qu'**il comprend au moins un gène rapporteur, les moyens permettant d'associer ledit gène rapporteur à une substance cible, lesdits moyens étant tels que lorsque la substance cible est une séquence d'acide nucléique cible, la transcription et la traduction dudit gène rapporteur ne conduisent pas à une protéine rapporteur fusionnée avec une protéine codée par ladite séquence d'acide nucléique cible, et les mélanges nécessaires à la transcription, la traduction et à la révélation de la protéine rapporteur codée par le gène rapporteur.

31. Un kit selon la revendication 30 pour la détection d'une séquence cible d'acide nucléique dans un échantillon, **caractérisé en ce qu'**il comprend un jeu d'amorces selon la revendication 24, un mélange nécessaire à l'amplification, les nucléotides triphosphates, une ARN polymérase ADN dépendante.

32. Un kit selon la revendication 30 la mise en oeuvre d'une méthode selon les revendications 18 à 20, **caractérisé en ce qu'**il comprend une sonde selon l'une des revendications 15 à 17, une ADN polymérase ADN dépendante, une ligase nick-sealing, les nucléotides triphosphates et les désoxynucléotides triphosphates, une amorce pour initier la réplication, une amorce permettant la synthèse du deuxième brin d'ADN, une ARN polymérase ADN dépendante, les mélanges nécessaires à la ligation, à la réplication.

33. Un kit selon la revendication 30 pour la mise en oeuvre de la méthode de détection d'une séquence cible dans un échantillon selon l'une des revendications 21 à 23 ou 26, **caractérisé en ce qu'**il comprend un jeu d'amorces selon l'une des revendications 25 ou 27 à 28, un mélange réactionnel d'amplification, éventuellement une ou plusieurs enzymes de restriction, une ARN polymérase ADN dépendante, une ADN polymérase ADN dépendante.

34. Une substance marquée par un gène rapporteur et les éléments nécessaires à l'expression in vitro dudit gène rapporteur, **caractérisé en ce que** ledit gène possède un promoteur d'ARN polymérase et éventuellement d'un terminateur d'ARN polymérase pour sa transcription et un site de fixation des ribosomes pour sa traduction, de façon à ce que lorsque la substance cible est une séquence d'acide nucléique cible, la transcription et la traduction dudit gène rapporteur ne conduisent pas à une protéine rapporteur fusionnée avec une protéine codée par ladite séquence d'acide nucléique cible.

## Patentansprüche

1. Methode zum Nachweis einer Zielsubstanz in einer Probe, **dadurch gekennzeichnet, dass** diese die folgenden Schritte umfasst:
a) die spezifische Markierung der besagten Substanz durch ein Reportergen und durch die zur *In-vitro*-Expression des besagten Reportergens notwendigen Sequenzen,
b) die In-vitro-Transkription und -Translation des besagten Reportergens in ein azelluläres System, wobei davon ausgegangen wird, dass, wenn die Zielsubstanz eine Nukleinsäurenzielsequenz ist, die Transkription und die Translation des besagten Reportergens nicht zu einem Reporterprotein führen, das mit einem durch die besagte Nukleinsäurenzielsequenz kodierten Protein fusioniert hat,
c) den *In-vitro*-Nachweis des durch das besagte Reportergen kodierten Reporterproteins.

2. Methode zum Nachweis einer Zielsubstanz in einer Probe nach Anspruch 1, **dadurch gekennzeichnet, dass** die Markierung von Schritt (a) darin besteht, die besagte markierte Substanz durch ein Reportergen zu erhalten, das mit einem RNA-Polymerase-Promoter und eventuell einem RNA-Polymerase-Terminator für seine Transkription und einer ribosomalen Bindungsstelle für seine Translation versehen ist, so dass in Schritt (b), wenn die Zielsubstanz eine Nukleinsäurenzielsequenz ist, die *In-vitro*-Transkription und -Translation des besagten Reportergens in ein azelluläres System nicht zu einem Reporterprotein führen, das mit einem durch die besagte Nukleinsäurenzielsequenz kodierten Protein fusioniert hat.

3. Methode zum Nachweis einer Zielsubstanz in einer Probe nach einem der Ansprüche 1 bis 2, **dadurch gekennzeichnet, dass** in Schritt (b) die Transkripte vor der Translation amplifiziert werden.

4. Methode zum Nachweis einer Zielsubstanz in einer Probe nach einem der Ansprüche 1 bis 3, **dadurch gekennzeichnet, dass** in Schritt (a) die Zielsubstanz mit dem Reportergen und den zur *In-vitro*-Expression des besagten Reportergens notwendigen Elementen direkt verbunden wird.

5. Methode zum Nachweis einer Zielsubstanz in einer Probe nach einem der Ansprüche 1 bis 3, **dadurch gekennzeichnet, dass** in Schritt (a) die Zielsubstanz mit dem Reportergen und den zur In-vitro-Expression des besagten Reportergens notwendigen Elementen mittels eines spezifischen Liganden der Zielsubstanz verbunden wird, wobei der besagte Ligand durch ein Reportergen und die zur *In-vitro*-Expression des besagten Reportergens notwendigen Sequenzen markiert wird.

6. Methode zum Nachweis einer Nukleinsäurenzielsequenz in einer Probe nach einem der Ansprüche 1 bis 5, **dadurch gekennzeichnet, dass** diese die folgenden Schritte umfasst:
a) die Präparierung, ausgehend von der Probe, eines rekombinanten Nukleinsäurenmoleküls, das von 5' nach 3' einen RNA-Polymerase-Promoter, ein Reportergen mit den zu seiner Expression notwendigen Sequenzen und eventuell einen RNA-Polymerase-Terminator umfasst, wobei die Nukleinsäurenzielsequenz zwischen zwei beliebigen dieser Elemente lokalisiert ist,
b) die Transkription und die Translation des in Schritt (a) erzeugten rekombinanten Nukleinsäuremoleküls, wobei die *In-vitro*-Transkription und -Translation des besagten rekombinanten Nukleinsäuremoleküls nicht zu einem Reporterprotein führen, das mit einem durch die besagte Nukleinsäurenzielsequenz kodierten Protein fusioniert hat,
c) die Aktivierung des durch das Reportergen kodierten, als Reportermolekül bezeichneten Proteins, das in Schritt (b) durch jedwedes geeignete Mittel erzeugt wurde.

7. Methode zum Nachweis einer Nukleinsäurenzielsequenz in einer Probe nach Anspruch 6, **dadurch gekennzeichnet, dass** das Nukleinsäuremolekül von Schritt (a) durch *In-vitro*-Amplifikation der Nukleinsäurenzielsequenz präpariert wird.

8. Methode zum Nachweis einer Nukleinsäurenzielsequenz in einer Probe nach Anspruch 7, **dadurch gekennzeichnet, dass** das Nukleinsäuremolekül von Schritt (a) durch Amplifikation mit Hilfe von zwei Primern präpariert wird:
- einem Sense-Primer, der mindestens einen Teil enthält, der Abschnitt 5' der Zielsequenz entspricht,
- einem Antisense-Primer, der mindestens einen Teil enthält, der Abschnitt 3' der Zielsequenz entspricht,
wobei die besagten Primer nach Amplifikation der Nukleinsäurenzielsequenz und nach Schritt (b) die Expression des Reportergens ermöglichen, um ein Reporterprotein zu erzeugen, das nicht mit einem durch die besagte Nukleinsäurenzielsequenz kodierten Protein fusioniert hat.

9. Methode zum Nachweis einer Nukleinsäurenzielsequenz in einer Probe nach einem der Ansprüche 7 oder 8, **dadurch gekennzeichnet, dass** Schritt (a) die drei folgenden Reaktionen umfasst:
a') die Amplifikation der Zielsequenz mit einem Primerpaar, wobei der Sense-Primer einen RNA-Polymerase-Promoter und der Antisense-Primer einen Abschnitt 5' trägt, der dem Anfang der Sequenz des Reportergens entspricht, und
a") die Inkontaktsetzung der Amplifikationsprodukte der vorhergehenden Reaktion mit dem Reportergen, das eventuell einen RNA-Polymerase-Terminator für seine Transkription und eine ribosomale Bindungsstelle für seine Translation trägt, so dass die besagten Amplifikationsprodukte mit dem besagten Reportergen hybridisieren, und schließlich
a''') die Amplifikation der Produkte von Schritt (a'') mit einem Primerpaar, wobei der Sense-Primer dem von Schritt (a') ähnlich ist und der Antisense-Primer einen Teil umfasst, der einem dem Reportergen nachgelagerten Abschnitt entspricht.

10. Methode zum Nachweis einer Nukleinsäurenzielsequenz in einer Probe nach einem der Ansprüche 7 oder 8, **dadurch gekennzeichnet, dass** Schritt (a) die zwei folgenden Reaktionen umfasst:
a') die Amplifikation der Zielsequenz mit einem Primerpaar, wobei der Sense-Primer einen RNA-Polymerase-Promoter und der Antisense-Primer einen Abschnitt 5' trägt, der dem Anfang der Sequenz des Reportergens entspricht, und
a'') die Amplifikation der Produkte von Schritt (a') mit einem Primerpaar, wobei der Sense-Primer dem von Schritt (a') gleich ist und der Antisense-Primer ein Mega-Primer ist, der aus dem Reportergen besteht, das eventuell einen RNA-Polymerase-Terminator für seine Transkription und eine ribosomale Bindungsstelle für seine Translation trägt.

11. Methode zum Nachweis einer Nukleinsäurenzielsequenz in einer Probe nach einem der Ansprüche 7 oder 8, **dadurch gekennzeichnet, dass** Schritt (a) die Amplifikationsreaktion der Zielsequenz mit einem Primerpaar umfasst, wobei der Sense-Primer einen RNA-Polymerase-Promoter und der Antisense-Primer einen Abschnitt 5' trägt, der eine kodierende Sequenz für eine ribosomale Bindungsstelle, ein Reportergen und eventuell einen Transkriptionsterminator umfasst.

12. Methode zum In-vitro-Nachweis einer Nukleinsäurenzielsequenz in einer Probe nach Anspruch 6, **dadurch gekennzeichnet, dass** die Präparierung des Nukleinsäuremoleküls von Schritt (a) durch Hybridisierung und Ligation einer Sonde vom Typ Padlock mit der Nukleinsäurenzielsequenz erfolgt.

13. Methode zum In-vitro-Nachweis einer Nukleinsäurenzielsequenz in einer Probe nach Anspruch 12, **dadurch gekennzeichnet, dass** die besagte Padlock-Sonde bei 3' und 5' aus Segmenten zusammengesetzt ist, die durch die Komplementärsequenz eines Reportergens getrennt sind, das die Komplementärsequenzen eines RNA-Polymerase-Promoters und eventuell eines -Terminators für seine *In-vitro*-Transkription und die Komplementärsequenz einer ribosomalen Bindungsstelle für seine In-vitro-Translation trägt.

14. Methode zum *In-vitro*-Nachweis einer Nukleinsäurenzielsequenz in einer Probe nach Anspruch 12, **dadurch gekennzeichnet, dass** die besagte Padlock-Sonde bei 5' und 3' aus Segmenten, die von 5' nach 3' durch die Sequenz eines RNA-Polymerase-Promoters getrennt sind, einer ribosomalen Bindungsstelle und der Sequenz eines Reportergens mit eventuell einem RNA-Polymerase-Terminator zusammengesetzt ist.

15. Eine Padlock-Sonde, die bei einer Methode nach einem der Ansprüche 12 bis 14 eingesetzt werden kann, **dadurch gekennzeichnet, dass** die besagte Sonde bei 5' und 3' aus Komplementärsequenzen der Nukleinsäurenzielsequenz zusammengesetzt ist, um mit dieser ein Verbindungshybrid auszubilden, wobei die besagten Segmente durch die Komplementärsequenz eines Reportergens getrennt sind, das die Komplementärsequenzen eines RNA-Polymerase-Promoters und eventuell eines -Terminators für seine *In-vitro*-Transkription und die Komplementärsequenz einer ribosomalen Bindungsstelle für seine In-vitro-Translation trägt, damit die In-vitro-Transkription und -Translation in ein azelluläres System des besagten Reportergens nicht zu einem Reporterprotein führen, das mit einem durch die besagte Nukleinsäurenzielsequenz kodierten Protein fusioniert hat.

16. Eine Padlock-Sonde, die bei einer Methode nach einem der Ansprüche 12 bis 14 eingesetzt werden kann, **dadurch gekennzeichnet, dass** die besagte Sonde bei 5' und 3' aus Komplementärsequenzen der Nukleinsäurenzielsequenz zusammengesetzt ist, um mit dieser ein Verbindungshybrid auszubilden, wobei die besagten Segmente durch die Sequenz eines RNA-Polymerase-Promoters, einer ribosomalen Bindungsstelle und der Sequenz eines Reportergens mit eventuell einem RNA-Polymerase-Terminator für seine *In-vitro*-Translation getrennt sind, damit die *In-vitro*-Transkription und -Translation in ein azelluläres System des besagten Reportergens nicht zu einem Reporterprotein führen, das mit einem durch die besagte Nukleinsäurenzielsequenz kodierten Protein fusioniert hat.

17. Eine Padlock-Sonde nach einem der Ansprüche 15 oder 16, **dadurch gekennzeichnet, dass** die Segmente eine einen mutierten Nukleotid tragende Nukleinsäurenzielsequenz ergänzen, um ein Hybrid auszubilden, bei dem sich der besagte mutierte Nukleotid am Verbindungspunkt der besagten hybridisierten Segmente auf der Nukleinsäurenzielsequenz befindet.

18. Methode zum In-vitro-Nachweis einer Nukleinsäurenzielsequenz in einer Probe nach einem der Ansprüche 12 bis 14, **dadurch gekennzeichnet, dass** die Sonde nach Zirkularisierung als Matrize für ihre Rollingcircle-Replikation eingesetzt wird.

19. Methode zum *In*-*vitro*-Nachweis einer Nukleinsäurenzielsequenz in einer Probe nach Anspruch 18, **dadurch gekennzeichnet, dass** die Rolling-Circle-Replikation mit Hilfe eines die Padlock-Sonde ergänzenden Primers, die zielkomplementär sind, durchgeführt wird, um die Replikation durch eine DNA-Polymerase zwecks Herstellung einer DNA-Matrize anzustoßen, die verknüpfte Reportergene mit allen notwendigen Signalen für ihre *In-vitro*-Expression trägt, und dadurch, dass der Komplementärstrang dieser DNA-Matrize ausgehend von einem zweiten Oligonukleotid-Primer synthetisiert wird, so dass diese Matrize doppelstrangig wird.

20. Methode zum *In-vitro*-Nachweis einer Nukleinsäurenzielsequenz in einer Probe nach einem der Ansprüche 12 bis 14 oder 18, **dadurch gekennzeichnet, dass** die Transkription des Reportergens auf die Padlock-Sonde nach ihrer Zirkularisierung und der Synthese des Komplementärstrangs mit Hilfe eines die Padlock-Sonde ergänzenden Primers, die zielkomplementär sind, direkt in Schritt (b) durchgeführt wird.

21. Methode zum *In-vitro*-Nachweis einer Nukleinsäurenzielsequenz in einer Probe nach einem der Ansprüche 6 bis 8, **dadurch gekennzeichnet, dass** die Nukleinsäurenzielsequenz von einer Nukleinsäurenprobe in Schritt (a) durch spezifische isothermische Amplifikation mit Hilfe einer DNA-abhängigen RNA-Polymerase und zweier spezifischer Primern der Zielsequenz isoliert wird, von denen mindestens einer von einem Teil 3' gebildet wird, der spezifisch mit der Zielsequenz hybridisieren kann, und einem Teil 5', der mindestens eine umgekehrt wiederholte Sequenz umfasst, um bei einer adäquaten Temperatur eine so genannte "Haarnadel"-Struktur auszubilden.

22. Methode zum *In-vitro*-Nachweis einer Nukleinsäurenzielsequenz in einer Probe nach Anspruch 21, **dadurch gekennzeichnet, dass** die beiden Primer eine ungekehrt wiederholte Sequenz tragen, um bei einer adäquaten Temperatur eine Haarnadel-Struktur auszubilden, wobei die beiden umgekehrt wiederholten Sequenzen identisch oder unterschiedlich sein können.

23. Methode zum *In-vitro*-Nachweis einer Nukleinsäurenzielsequenz in einer Probe nach einem der Ansprüche 21 oder 22, **dadurch gekennzeichnet, dass** Schritt (a) der isothermischen Amplifikation die folgenden Schritte umfasst:
i) man erhitzt ein Reaktionsgemisch, die Nukleinsäurenprobe umfassend, in der die Zielsequenz eventuell vorhanden ist, wobei die vier Deoxynukleotid-Triphosphate, Salze und Reagenzien eine optimale Aktivität der DNA-Polymerase gewährleisten, eine DNA-abhängige DNA-Polymerase, wenn diese thermophil ist (ansonsten iii), ein spezielles Primerpaar für die zu amplifizierende Zielsequenz mit den umgekehrt wiederholten Sequenzen zur Separierung der DNA-Stränge, danach
ii) führt man eine Abkühlung herbei, um die Hybridisierung der Primer zu ermöglichen, und
iii) man fügt DNA-Polymerase hinzu, falls diese mesophil ist, danach
iv) setzt man das Reaktionsgemisch der der Elongation durch die DNA-Polymerase adäquaten Temperatur aus,
v) man wiederholt ein zweites Mal die Schritte Erhitzung (i), Hybridation (ii), Hinzufügung der DNA-Polymerase, sofern diese mesophil ist (iii), und Elongation (iv), um eine Zielsequenz zu erhalten, deren Enden aus umgekehrt wiederholten Sequenzen bestehen,
vi) man lässt die Amplifikationsreaktion ablaufen, bei der die Produkte des vorhergehenden Schritts Matrize und Primer sind, bei konstanter Temperatur, die so gewählt ist, dass gewährleistet ist:
- das Gleichgewicht der umgekehrt wiederholten Sequenzen zwischen der Linear- und Haarnadelform,
- die Elongation der Primer durch die DNA-abhängige DNA-Polymerase.

24. Ein Primersatz zur Amplifikation einer Nukleinsäurenzielsequenz, der nach einem der Ansprüche 7 bis 11 und 21 bis 23 der Methode gemäß eingesetzt werden kann, **dadurch gekennzeichnet, dass** dieser umfasst:
- einen Sense-Primer, umfassend mindestens einen Teil, der Abschnitt 5' der Zielsequenz entspricht,
- einen Antisense-Primer, umfassend mindestens einen Teil, der Abschnitt 3' der Zielsequenz entspricht,
wobei die besagten Primer so beschaffen sind, dass:
- man nach Amplifikation der Nukleinsäurenzielsequenz ein rekombinantes Nukleinsäuremolekül erhält, umfassend in Richtung 5' nach 3' einen RNA-Polymerase-Promoter, ein Reportergen mit den zu seiner In-vitro-Expression notwendigen Sequenzen und eventuell einen RNA-Polymerase-Terminator, und
- die In-vitro-Transkription und -Translation des besagten Reportergens in ein azelluläres System nicht zu einem Reporterprotein führen, das mit einem durch die besagte Nukleinsäurenzielsequenz kodierten Protein fusioniert hat.

25. Ein Primersatz nach Anspruch 24, **dadurch gekennzeichnet, dass** mindestens einer der besagten Primer eine bei 5' umgekehrt wiederholte Sequenz umfasst.

26. Methode zum *In-vitro*-Nachweis einer Nukleinsäurenzielsequenz in einer Probe nach einem der Ansprüche 21 bis 23, **dadurch gekennzeichnet, dass** die Amplifikationsprodukte getrennt werden können, speziell durch ein Restriktionsenzym, während oder nach der Reaktion zur Amplifikation der Zielsequenz.

27. Ein Primersatz nach Anspruch 25, der nach Anspruch 26 der Methode gemäß eingesetzt werden kann, **dadurch gekennzeichnet, dass** mindestens einer der beiden Primer eine Restriktionsstelle umfasst, und wenn beide eine Restriktionsstelle tragen, können diese identisch oder unterschiedlich sein.

28. Ein Primersatz nach Anspruch 27, **dadurch gekennzeichnet, dass** sich die auf mindestens einem der Primer vorhandene Restriktionsstelle in einem Ring einer Haarnadel befindet, genauer zwischen zwei umgekehrt wiederholten Sequenzen einer der Primer.

29. Einsatz eines Reportergens und der zu seiner *In-vitro*-Expression in einem azellulären System notwendigen Elemente zur spezifischen Markierung einer in einer Probe enthaltenen Zielsubstanz, wobei die besagte Markierung so vonstatten geht, dass, wenn die Zielsequenz eine Nukleinsäurenzielsequenz ist, die Transkription und die Translation des besagten Reportergens nicht zu einem Reporterprotein führen, das mit einem durch die besagte Nukleinsäurenzielsequenz kodierten Protein fusioniert hat.

30. Ein Kit zur Durchführung der Methode zum Nachweis einer Zielsequenz nach einem der Ansprüche 1 bis 6, **dadurch gekennzeichnet, dass** dieses mindestens ein Reportergen umfasst, die Mittel zur Verbindung des besagten Reportergens mit einer Zielsubstanz, wobei die besagten Mittel dergestalt sind, dass, wenn die Zielsubstanz eine Nukleinsäurenzielsequenz ist, die Transkription und die Translation des besagten Reportergens nicht zu einem Reporterprotein führen, das mit einem durch die besagte Nukleinsäurenzielsequenz kodierten Protein fusioniert hat, und die zur Transkription, Translation und Offenbarung des durch das Reportergen kodierten Reporterproteins notwendigen Mischungen.

31. Ein Kit nach Anspruch 30 zum Nachweis einer Nukleinsäurenzielsequenz in einer Probe, **dadurch gekennzeichnet, dass** dieses einen Primersatz nach Anspruch 24 umfasst, eine für die Amplifikation notwendige Mischung, die Nukleotid-Triphosphate, eine DNA-abhängige RNA-Polymerase.

32. Ein Kit nach Anspruch 30 zur Durchführung einer Methode nach den Ansprüchen 18 bis 20, **dadurch gekennzeichnet, dass** dieses eine Sonde nach einem der Ansprüche 15 bis 17 umfasst, eine DNA-abhängige DNA-Polymerase, eine Nick-sealing-Ligase, die Nukleotid-Triphosphate und die Deoxynukleotid-Triphosphate, einen Primer zum Anstoss der Replikation, einen Primer für die Synthese des zweiten DNA-Strangs, eine DNA-abhängige RNA-Polymerase, die zur Ligation und zur Replikation notwendigen Mischungen.

33. Ein Kit nach Anspruch 30 zur Durchführung der Methode zum Nachweis einer Nukleinsäurenzielsequenz in einer Probe nach einem der Ansprüche 21 bis 23 oder 26, **dadurch gekennzeichnet, dass** dieses einen Primersatz nach einem der Ansprüche 25 oder 27 bis 28 umfasst, ein Reaktionsgemisch zur Amplifikation, eventuell ein oder mehrere Restriktionsenzyme, eine DNA-abhängige RNA-Polymerase, eine DNA-abhängige DNA-Polymerase.

34. Eine von einem Reportergen markierte Substanz und die zur *In-vitro*-Expression des besagten Reportergens notwendigen Elemente, **dadurch gekennzeichnet, dass** das besagte Gen einen RNA-Polymerase-Promoter und eventuell einen RNA-Polymerase-Terminator für seine Transkription und eine ribosomale Bindungsstelle für seine Translation trägt, so dass, wenn die Zielsubstanz eine Nukleinsäurenzielsequenz ist, die Transkription und die Translation des besagten Reportergens nicht zu einem Reporterprotein führen, das mit einem durch die besagte Nukleinsäurenzielsequenz kodierten Protein fusioniert hat.

## Claims

1. A method for detecting a target substance in a sample, **characterized in that** it comprises the following steps:
a) specific marking of said substance by a reporter gene and by the sequences required for expressing said reporter gene *in vitro,*
b) *in vitro* transcription and translation in an acellular system of said reporter gene, it being understood that when the target substance is a target nucleic acid sequence, the transcription and translation of said reporter gene does not lead to a reporter protein fused with a protein coded by said target nucleic acid sequence,
c) *in vitro* detection of the reporter protein coded by said reporter gene.

2. The method for detecting a target substance in a sample according to claim 1, **characterized in that** the marking of step (a) consists of obtaining said substance marked by a reporter gene provided with a RNA polymerase promoter and possibly with a RNA polymerase terminator for its transcription and a site for fixing the ribosomes for its translation, so that in step (b), when the target substance is a target nucleic acid sequence, the *in vitro* transcription and translation in an acellular system of said reporter gene, does not lead to a reporter protein fused with a protein coded by said target nucleic acid sequence.

3. The method for detecting a target substance in a sample according to any of claims 1 to 2, **characterized in that** in step (b), a step for amplifying the transcripts is carried out before the translation.

4. The method for detecting a target substance in a sample according to any of claims 1 to 3, **characterized in that** in step (a), the target substance is directly associated with the reporter gene and with elements required for expressing said reporter gene *in vitro.*

5. The method for detecting a target substance in a sample according to any of claims 1 to 3, **characterized in that** in step (a), the target substance is associated with the reporter gene and with the sequences required for its expression *in vitro,* via a specific ligand of the target substance, said ligand being marked by a reporter gene and by the sequences required for expressing said reporter gene *in vitro*.

6. The method for detecting a target nucleic acid sequence in a sample according to any of claims 1 to 5, **characterized in that** it comprises the following steps:
a) preparation from the sample, of a recombinant nucleic acid molecule comprising in the 5' to 3' direction, a RNA polymerase promoter, a reporter gene, having the sequences required for its expression, and possibly a RNA polymerase terminator, the target nucleic acid sequence being localized between any two of these elements,
b) transcription and translation of the recombinant nucleic acid molecule obtained in step (a), the *in vitro* transcription and translation of said recombinant nucleic acid molecule not leading to a reporter protein fused with a protein coded by said target nucleic acid sequence,
c) revelation of the activity of the protein coded by the reporter gene, referred to as the reporter molecule, obtained in step (b) by any suitable means.

7. The method for detecting a target nucleic acid sequence in a sample according to claim 6, **characterized in that** the preparation of the nucleic acid molecule of step (a) is performed by amplification of the target nucleic acid sequence in vitro.

8. The method for detecting a target nucleic acid sequence in a sample according to claim 7, **characterized in that** the preparation of the nucleic acid molecule of step (a) is performed by amplification by means of two primers:
- a sense primer comprising at least a portion homologous to region 5' of the target sequence,
- an antisense primer comprising at least a portion homologous to region 3' of the target sequence,
said primers allowing, after amplification of the target nucleic acid sequence and after step (b), the reporter gene to be expressed in order to obtain a reporter protein non-fused with a protein coded by said target nucleic acid sequence.

9. The method for detecting a target nucleic acid sequence in a sample according to any of claims 7 or 8, **characterized in that** step (a) comprises the three following reactions:
a') amplification of the target sequence with a pair of primers with the sense primer having a RNA polymerase promoter and with the antisense primer having a 5' region homologous to the beginning of the sequence of the reporter gene, and
a'') bringing the amplification products of the previous reaction in contact with the reporter gene possibly having a RNA polymerase terminator for its transcription, a site for fixing ribosomes for its translation, so as to hybridize said amplification products with said reporter gene, and then
a''') amplification of the products from step (a'') with a pair of primers, the sense primer of which is similar to the one of step (a') and the antisense primer of which comprises a portion homologous to a region downstream from the reporter gene.

10. The method for detecting a target nucleic acid sequence in a sample according to any of claims 7 or 8, **characterized in that** step (a) comprises the following two reactions:
a') amplification of the target sequence with a pair of primers, the sense primer of which has a RNA polymerase promoter and the antisense primer of which has a 5' region homologous to the beginning of the sequence of the reporter gene, and
a'') amplification of the products from step (a') with a pair of primers, the sense primer of which is identical with the one in step (a') and the antisense primer of which is a megaprimer consisting of the reporter gene possibly having a RNA polymerase terminator for its transcription, a site for fixing ribosomes for its translation.

11. The method for detecting a target nucleic acid sequence in a sample according to any of claims 7 or 8, **characterized in that** step (a) comprises the reaction for amplifying the target sequence with a pair of primers, the sense primer of which has a RNA polymerase promoter and the antisense primer of which has a 5' region comprising a sequence coding for a site for fixing ribosomes, a reporter gene and possibly a transcription terminator.

12. The method for detecting a target nucleic acid sequence in a sample *in vitro* according to claim 6, **characterized in that** the preparation of the nucleic acid molecule of step (a) is performed by hybridization and ligation of a probe of the padlock type with the target nucleic acid sequence.

13. The method for detecting a target nucleic acid sequence in a sample according to claim 12, **characterized in that** said padlock probe consists of segments in 3' and 5' separated by the complementary sequence of a reporter gene which has the complementary sequences of a promoter and possibly of a RNA polymerase terminator for its transcription, and the complementary sequence of a site for fixing ribosomes for its translation *in vitro.*

14. The method for detecting a target nucleic acid sequence in a sample *in vitro* according to claim 12, **characterized in that** said probe consists of segments in 5' and 3', separated from 5' in 3' by the sequence of a RNA polymerase promoter, of a site for fixing ribosomes and of the sequence of a reporter gene with possibly a RNA polymerase terminator.

15. A padlock probe able to be used in a method according to any of claims 12 to 14, **characterized in that** said probe consists, in 5' and 3', of complementary segments of the target nucleic acid sequence so as to form with the latter a contiguous hybrid, said segment being separated by the complementary sequence of a reporter gene which has the complementary sequences of a promoter and possibly of a RNA polymerase terminator for its transcription, and the complementary sequence of a site for fixing ribosomes for its translation *in vitro,* so that the *in vitro* transcription and translation in an acellular system of said reporter gene, does not lead to a reporter protein fused with a protein coded by said target nucleic acid sequence.

16. A padlock probe able to be used in a method according to any of claims 12 to 14, **characterized in that** said probe consists, in 5' and 3', of complementary segments of the target nucleic acid sequence, so as to form with the latter a contiguous hybrid, said segments being separated by the sequence of a RNA polymerase promoter, of a site for fixing ribosomes and of the sequence of a reporter gene with possibly a RNA polymerase terminator for its translation *in* *vitro* so that the *in vitro* transcription and translation in an acellular system of the reporter gene does not lead to a reporter protein fused with a protein coded by said target nucleic acid sequence.

17. A padlock probe according to any of claims 15 or 16, **characterized in that** the segments are complementary to a target nucleic acid sequence bearing a mutated nucleotide so as to form a hybrid wherein said mutated nucleotide is found at the junction point of said hybridized segments on the target nucleic acid sequence.

18. The method for detecting a target nucleic acid sequence in a sample *in vitro* according to any of claims 12 to 14, **characterized in that** after annealing, the probe is used as a matrix for its rolling circle replication.

19. The method for detecting a target nucleic acid sequence in a sample *in vitro* according to claim 18, **characterized in that** the rolling circle replication is performed by a complementary primer to the padlock probe complementary of the target for initiating replication by a DNA polymerase, so as to produce a DNA matrix having a series of reporter genes with all the signals required for its expression *in vitro,* and **in that** the complementary strand of this DNA matrix is synthesized from a second oligonucleotide primer so as to make this matrix double-stranded.

20. The method for detecting a target nucleic acid sequence in a sample *in vitro* according to any of claims 12 to 14 or 18, **characterized in that** the transcription of the reporter gene is directly performed in step (b) on the padlock probe after its annealing and synthesis of the complementary strand by a complementary primer to the padlock probe complementary of the target.

21. The method for detecting a target nucleic acid sequence in a sample *in vitro* according to any of claims 6 to 8, **characterized in that** the target nucleic acid sequence is isolated from a nucleic acid sample in step (a) by specific isothermal amplification by means of a DNA-dependent DNA polymerase and two specific primers of the target sequence, one of which at least consists of a portion 3' which may specifically hybridize with the target sequence and of a portion 5' including at least an inverted repeated sequence for forming a so-called "hairpin" structure at an adequate temperature.

22. The method for detecting a target nucleic acid sequence in a sample *in vitro* according to claim 21, **characterized in that** both primers have an inverted repeated sequence for forming a hairpin structure at an adequate temperature, wherein said inverted repeated sequences of both primers may be identical or different.

23. The method for detecting a target nucleic acid sequence in a sample *in vitro* according to any of claims 21 or 22, **characterized in that** the isothermal amplification step (a) comprises the following steps:
i) a reaction mixture is heated, which comprises the sample of nucleic acid in which the target sequence is possibly present, the four triphosphate deoxynucleotides, salts and reagents providing optimal activity of the DNA polymerase, a DNA-dependent DNA polymerase if the latter is thermophilic, (else iii), a pair of specific primers of the target sequence to be amplified and comprising the repeated inverted sequences in order to separate the DNA strands, and then
ii) it is cooled in order to enable hybridization of the primers, and
iii) DNA polymerase is added if the latter is mesophilic, and then
iv) the reaction mixture is placed at an adequate temperature for elongation by the DNA polymerase,
v) the steps for heating (i), hybridization (ii), adding DNA polymerase if it is mesophilic (iii), and for elongation (iv) are repeated a second time in order to obtain a target sequence with ends consisting of inverted repeated sequences,
vi) the amplification reaction is allowed to proceed with the products of the previous steps as matrix and primer, at a constant temperature selected in order to provide:
- equilibrium of the inverted repeated sequences between the linear form and the hairpin form,
- elongation of the primers by the DNA-dependent DNA polymerase.

24. A set of primers for amplifying a target nucleic acid sequence, able to be used in the method according to any of claims 7 to 11 and 21 to 23, **characterized in that** it comprises:
- a sense primer at least comprising a portion. homologous to the 5' region of the target sequence,
- an antisense primer, at least comprising a portion homologous to the 3' region of the target sequence,
said primers being such that:
- after amplification of the target nucleic acid sequence, a recombinant nucleic acid molecule is obtained, comprising in the 5' to 3' direction, a RNA polymerase promoter, a reporter gene having the sequences required for its expression *in vitro,* and possibly a RNA polymerase terminator, and
- transcription and translation of said reporter gene in vitro in an acellular system does not lead to a reporter protein fused with a protein coded by said target nucleic acid sequence.

25. A set of primers according to claim 24, **characterized in that** at least one of said primers includes an inverted repeated sequence in 5'.

26. The method for detecting a target nucleic acid sequence in a sample in vitro according to any of claims 21 to 23, **characterized in that** the amplification product may be specifically cut by a restriction enzyme, during or after the reaction for amplifying the target sequence.

27. A set of primers according to claim 25, able to be used in the method according to claim 26, **characterized in that** at least one of both primers includes a restriction site and if both of them have a restriction site, the latter may be identical or different.

28. A set of primers according to claim 27, **characterized in that** the restriction site present on at least one of the primers is located in a loop of a hairpin, more specifically between two inverted repeated sequences of one of the primers.

29. The use of a reporter gene and of the element required for its expression *in vitro,* in an acellular system for specifically marking a target substance contained in a sample, said marking being such that, when the target substance is a target nucleic acid sequence, the transcription and translation of said reporter gene does not lead to a reporter protein fused with a protein coded by said target nucleic acid sequence.

30. A kit for implementing the method for detecting a target substance according to any of claims 1 to 6, **characterized in that** it comprises at least a reporter gene, the means enabling said reporter gene to be associated with a target substance, said means being such that, when the target substance is a target nucleic acid sequence, the transcription and translation of said reporter gene does not lead to a reporter protein fused with a protein coded by said target nucleic acid sequence, and the mixtures required for the transcription, translation, and revelation of the reporter protein coded by the reporter gene.

31. A kit according to claim 30 for detecting a nucleic acid target sequence in a sample, **characterized in that** it comprises a set of primers according to claim 24, a mixture required for amplification, triphosphate nucleotides, a DNA-dependent RNA polymerase.

32. A kit according to claim 30 for implementing a method according to claims 18 to 20, **characterized in that** it comprises a probe according to any of claims 15 to 17, a DNA-dependent DNA polymerase, a nick-sealing ligase, triphosphate nucleotides, and triphosphate deoxynucleotides, a primer for initiating the replication, a primer allowing the synthesis of the second DNA strand, a DNA-dependent RNA polymerase, the mixtures required for ligation, replication.

33. A kit according to claim 30 for implementing the method for detecting a target sequence in a sample, according to any of claims 21 to 23 or 26, **characterized in that** it comprises a set of primers according to any of claims 25 or 27 to 28, an amplification reaction mixture, possibly one or several restriction enzymes, a DNA-dependent RNA polymerase, a DNA-dependent DNA polymerase.

34. A substance marked by a reporter gene and elements required for the in vitro expression of said reporter gene, **characterized in that** said gene has a RNA polymerase promoter and possibly a RNA polymerase terminator for its transcription and a site for fixing ribosomes for its translation, so that, when the target substance is a target nucleic acid sequence, the transcription and translation of said reporter gene does not lead to a reporter protein fused with a protein coded by said target nucleic acid sequence.
